# EUROPEAN PATENT SPECIFICATION

(11) **EP 2 926 773 B1**
(45) Date of publication and mention of the grant of the patent: **23.08.2017**
(21) Application number: 15156271.7
(22) Date of filing: 20.09.2013
(51) Int. Cl.: A61F 2/30, A61F 2/38

(54) **Orthopaedic knee prosthesis system**
Orthopädisches Knieprothesensystem
Système de prothèse de genou orthopédique

(30) Priority: 20.09.2012 US 201261703404 P; 15.03.2013 US 201313832439
(43) Date of publication of application: 07.10.2015
(62) Divisional of application: 13185436.6
(73) Proprietor: DePuy Ireland Unlimited Company, County Cork (IE)
(72) Inventor: Bernasek, Thomas L, Tampa, FL Florida 33609 (US); Crossett, Lawrence S, Pittsburgh, PA Pennsylvania 15238 (US); Haidukewych, George J, Orlando, FL Florida 32819 (US); Lieberman, Jay R, Avon, CT Connecticut 06001 (US); Sordelet, Benjamin J, Columbia City, IN Indiana 46725 (US); Jones, Richard Spencer, Yockleton, Shropshire SY5 9PY (GB); Vendrely, Timothy G, Fort Wayne, IN Indiana 46805 (US); Wainscott, Stephanie M, Warsaw, IN Indiana 46580 (US); Cook, Michael A, Warsaw, IN Indiana 46581 (US); Chaney, Rebecca L, Warsaw, IN Indiana 46581 (US); WYSS, Joseph G, Warsaw, IN Indiana 46581 (US); HAZEBROUCK, Stephen A, Warsaw, IN Indiana 46581 (US); MATYAS, Aaron J, Warsaw, IN Indiana 46581 (US)
(74) Representative: Curran, Clair

(56) References cited:
- US-A- 6 071 311
- US-A1- 2004 049 285
- US-A1- 2012 016 482
- US-B2- 7 799 085

## Description

The present invention relates generally to prosthetic joints, and more particularly to a modular prosthetic knee joint system that includes a metaphyseal sleeve component.

The knee joint basically consists of the bone interface of the distal end of the femur and the proximal end of the tibia. Appearing to cover or at least partially protect this interface is the patella, which is a sesamoid bone within the tendon of the long muscle (quadriceps) on the front of the thigh. This tendon inserts into the tibial tuberosity and the posterior surface of the patella is smooth and glides over the femur.

The femur is configured with two knob like processes (the medial condyle and the lateral condyle) which are substantially smooth and which articulate with the medial plateau and the lateral plateau of the tibia, respectively. The plateaus of the tibia are substantially smooth and slightly cupped thereby providing a slight receptacle for receipt of the femoral condyles.

When the knee joint is damaged whether as a result of an accident or illness, a prosthetic replacement of the damaged joint may be necessary to relieve pain and to restore normal use to the joint. Typically the entire knee joint is replaced by means of a surgical procedure that involves removal of the surfaces of the corresponding damaged bones and replacement of these surfaces with prosthetic implants. This replacement of a native joint with a prosthetic joint is referred to as a primary total-knee arthroplasty.

On occasion, the primary knee prostheses fails. Failure can result from many causes, including wear, aseptic loosening, osteolysis, ligamentous instability, arthrofibrosis and patellofemoral complications. When the failure is debilitating, revision knee surgery may be necessary. In a revision, the primary knee prosthesis is removed and replaced with components of a revision prosthetic knee system.

Knee implant systems for both primary and revision applications are available from a variety of manufacturers, including DePuy Synthes. DePuy Synthes and others offer several different systems for both primary and revision applications. For example, DePuy Synthes offers systems under the trade marks PFC SIGMA, LCS and S-ROM. These orthopaedic knee systems includes several components, some appropriate for use in primary knee arthroplasty and some appropriate for use in revision surgery.

DePuy Synthes also offers other orthopaedic implant systems for other applications. One such system is the system sold under the trade mark LPS. The LPS system is provided for use in cases of severe trauma and disease. In such cases, the trauma or disease can lead to significant amounts of bone loss. The LPS system provides components that can replace all or significant portions of a particular bone, such as the femur. The DePuy Synthes LPS system is described more fully in EP-A-1358860.

In some patients, the metaphysis of the bone near the joint presents cavitary defects that are not completely filled by standard knee implants. The presence of such metaphyseal defects can result in loosening of the prosthetic implant over time, compromising the stability of the prosthetic implant and frequently requiring revision of the prosthetic implant.

To fill metaphyseal cavitary defects, knee systems with modular metaphyseal sleeves have been provided. Such sleeves are disclosed in, for example, US-A-2010/ 114323, US-A-2006/030945, US-7799085, US-7291174, US-6171342, US-5824097, US-5782921 and US-4634444. Such sleeves have been used in commercially available prosthetic knee implant systems, such as those sold under the trade marks PFC SIGMA, LCS, S-ROM and LPS.

Modular sleeves have also been used in hip implant systems, as disclosed in, for example, US-6264699, US-4790852. Such hip sleeves have been used in commercially available prosthetic hip implant systems, such as the S-ROM hip systems, available from DePuy Synthes.

In knee systems with modular metaphyseal sleeves, the conventional shape of many of the sleeves is generally an elliptical cone with a large ellipse profile close to the joint line tapering down to a smaller elliptical or circular profile at the termination of the component distal to the joint line. Generally, the sleeves have a terraced or stepped outer surface and an inner channel for frictional fixation to another component. This geometry fills cavitary defects in the metaphysis, allows for a wider surface area for load transfer through the joint and provides rotational stability for the articulating components of the prosthesis.

The outer surface of the sleeve is supported by solid bony structure or the bone bed. In the case of the distal femur, patient anatomy and the condition of the bone, particularly in a revision surgery, may require that the distal femur be resected to a more proximal level. Implanting a prosthetic distal femoral component and sleeve at this more proximal level may elevate the joint line (that is, the line defined by the articulation of the articular surfaces of the distal femoral component and proximal tibial component). Elevation of the joint line may adversely affect performance of the prosthetic knee system: the positions of the collateral ligament attachments to the femur relative to the joint line may impact knee kinematics, the articulation of the patella against the femoral component will be impacted, and the function of the extensor mechanism will also be impacted.

Prosthetic knee implant systems have commonly included femoral augments for use on the distal and posterior bone-facing surfaces of the femoral implant components. Examples of such augments are disclosed in US-6005018 and US-5984969. Such components serve to augment the inferior and posterior portions of the femoral component to add additional thickness to compensate for the lack of sufficient boney tissue, allowing the joint line to be distalized. However, with the femoral component so distalized, the metaphyseal sleeve used with the femoral component may no longer be optimally seated on a healthy bone bed. To compensate, surgeons may sometimes opt to use a larger size of metaphyseal sleeve.

US-7799085 discloses a modular knee implant system in which the femoral component can be fitted with one of a number of metaphyseal components. The metaphyseal components have stepped outer surfaces. The different components have different sizes, differing in their lengths and in their widths (measured in the medial-lateral direction).

Accordingly, a need exists for a knee prosthesis system that allows the surgeon the flexibility to optimize the position of the joint line while also allowing for a metaphyseal sleeve to be optimally sized and optimally positioned on a healthy bone bed.

A modular knee implant system that allows the surgeon to prepare the bone to receive a metaphyseal sleeve and to optimize the position of the joint line without further bone preparation to receive a different size of metaphyseal sleeve is provided.

The invention provides an orthopaedic knee prosthesis system as defined in claim 1.

Optionally, the first outer geometry is defined by a plurality of stepped walls and a plurality of annular walls, each annular wall connecting a pair of stepped walls of the plurality of stepped walls.

Optionally, the plurality of stepped walls defining the first outer geometry is a first plurality of stepped walls, and the second outer geometry is defined by a second plurality of stepped walls and a second plurality of annular walls, each annular wall of the second plurality of annular walls connecting a pair of stepped walls of the second plurality of stepped walls.

Optionally, each stepped wall of the first plurality of stepped walls has a maximum medial-lateral dimension, each stepped wall of the second plurality of stepped walls has a maximum medial-lateral dimension, and the maximum medial-lateral dimension of each stepped wall of the first plurality of stepped walls is less than the maximum medial-lateral dimension of each stepped wall of the second plurality of stepped walls.

Optionally, each stepped wall of the first plurality of stepped walls has a maximum anterior-posterior dimension, each stepped wall of the second plurality of stepped walls has a maximum anterior-posterior dimension, and the maximum anterior-posterior dimension of each stepped wall of the first plurality of stepped walls is less than the maximum anterior-posterior dimension of each stepped wall of the second plurality of stepped walls.

Optionally, the orthopaedic knee prosthesis system includes a third sleeve component which includes (i) a first end configured to be separately secured to the prosthetic knee component in place of the first sleeve component, (ii) a first tapered section extending distally from a second end positioned opposite the first end, (iii) a second tapered section extending distally from the first tapered section, and (iv) a third tapered section extending distally from the first tapered section, in which the second tapered section of the third sleeve component has the second outer geometry and the third tapered section has a third outer geometry different from the first outer geometry and the second outer geometry.

Optionally, the tapered section of the first sleeve component defines a maximum axial length of the first sleeve component, the first tapered section and the second tapered section of the second sleeve component define a maximum axial length of the second sleeve component that is greater than the maximum axial length of the first sleeve component, the first tapered section, the second tapered section, and the third tapered section of the third sleeve component define a maximum axial length of the third sleeve component that is greater than the maximum axial length of the second sleeve component.

Optionally, the first tapered section of the third sleeve component has the first outer geometry.

A modular knee prosthesis system can include a distal femoral implant component, a proximal tibial implant component and two metaphyseal members. The distal femoral implant component has a pair of spaced, curved distal condylar surfaces and a stem. The stem has an outer surface tapering from a distal end in the proximal direction. The outer surface of the stem has a maximum outer diameter at the distal end and a smaller outer diameter at a second position proximal to the distal end. The proximal tibial implant component has an articulating surface to receive and articulate with the distal articulating surfaces of the distal femoral component and a stem. The tibial stem has an outer surface tapering from a proximal end in the distal direction. The outer surface of the tibial stem has a maximum outer diameter at the proximal end and a smaller outer diameter at a second position distal to the proximal end. The first metaphyseal member has an outer surface that tapers in a proximal direction and an inner surface defining a tapered bore sized and shaped to be mountable on the stem of one of the implant components and to create a frictional lock between the stem and the first metaphyseal member. The outer surface of the first metaphyseal member comprises a stepped portion having a plurality of steps. Each step has a maximum medial-lateral dimension and a maximum anterior-posterior dimension. The stepped portion of the first metaphyseal sleeve has an overall axial length L. The second metaphyseal member has an outer surface that tapers in a proximal direction and an inner surface defining a tapered bore sized and shaped to be mountable on the stem of one of the implant components and to create a frictional lock between the stem component and the second metaphyseal member. The outer surface of the second metaphyseal member comprises a stepped portion having a plurality of steps. Each step has a maximum medial-lateral dimension and a maximum anterior-posterior dimension. The stepped portion of the second metaphyseal sleeve has an overall axial length L+X and has a common geometry with the first metaphyseal sleeve over axial length L: over axial length L, the maximum medial-lateral dimension and maximum anterior-posterior dimension of each step is the same as the maximum medial-lateral dimension and maximum anterior-posterior dimension of each step over the axial length L of the stepped portion of the first metaphyseal member. With this common geometry over axial length L, the same prepared bone space will receive either the first metaphyseal member or the second metaphyseal member.

Optionally, the first metaphyseal member and the second metaphyseal member have the same number of steps over axial length L of the stepped portions of the first metaphyseal member and the second metaphyseal member.

Optionally, each step of the stepped portion of the first metaphyseal member has an axial height, each step of the stepped portion of the second metaphyseal member has an axial height, and the axial heights of corresponding steps of the first metaphyseal member and the second metaphyseal member are the same.

Optionally, the system includes a third metaphyseal member having an outer surface that tapers in a proximal direction and an inner surface defining a tapered bore sized and shaped to be mountable on the stem of one of the implants components and to create a frictional lock between the stem and the third metaphyseal member. The outer surface of the third metaphyseal member comprises a stepped portion having a plurality of steps. Each step has a maximum medial-lateral dimension and a maximum anterior-posterior dimension. The stepped portion having an overall axial length L+X+Y. The maximum medial-lateral dimension and maximum anterior-posterior dimension of each step over the axial length L of the stepped portion of the third metaphyseal member is the same as the maximum medial-lateral dimension and maximum anterior-posterior dimension of each step over the axial length L of the stepped portion of the first metaphyseal member and the second metaphyseal member. The maximum medial-lateral dimension and maximum anterior-posterior dimension of each step over the axial length L+X of the stepped portion of the third metaphyseal member is the same as the maximum medial-lateral dimension and maximum anterior-posterior dimension of each step over the axial length L+X of the stepped portion of the second metaphyseal member.

Optionally, the tapered bore of the first metaphyseal member is sized and shaped to be mountable on the stem of the distal femoral implant component and to create a frictional lock between the stem of the distal femoral implant component and the first metaphyseal member and the tapered bore of the second metaphyseal member is sized and shaped to be mountable on the stem of the distal femoral implant component and to create a frictional lock between the stem of the distal femoral implant component and the first metaphyseal member. The contact between the articulating surfaces of the tibial member and the distal femoral component define a first joint line when the distal femoral component is assembled with the first metaphyseal member and the contact between the articulating surfaces of the tibial member and the distal femoral component define a second joint line when the distal femoral component is assembled with the second metaphyseal member. The second joint line is more distal than the first joint line in this construction.

Optionally, the distance between the first joint line and the second joint line corresponds with the difference between the overall axial lengths of the first metaphyseal member and the second metaphyseal member and defines a distal offset.

A modular knee prosthesis system can comprise a distal femoral component, a first metaphyseal member and a second metaphyseal member. The distal femoral component has a pair of spaced, curved distal condylar surfaces and a stem having an outer surface tapering from a distal end in the proximal direction. The outer surface of the femoral stem has a maximum outer diameter at the distal end and a smaller outer diameter at a second position proximal to the distal end. The first metaphyseal member includes an inner surface defining a tapered bore sized and shaped to be mountable on the stem of the distal femoral component and to create a frictional lock between the stem of the distal femoral component and the first metaphyseal member. The tapered bore extends proximally from an opening at the distal end of the first metaphyseal member. The first metaphyseal member also includes a tapered stepped outer surface having an axial length L. The second metaphyseal member includes an inner surface defining a tapered bore sized and shaped to be mountable on the stem of the distal femoral component and to create a frictional lock between the stem of the distal femoral component and the second metaphyseal member, the tapered bore extending proximally from an opening at the distal end of the second metaphyseal member. The second metaphyseal member includes a tapered stepped outer surface having an axial length L+X. The tapered stepped outer surface of the first metaphyseal member and the tapered stepped outer surface of the second metaphyseal member have the same shape and the same medial-lateral dimensions and the same anterior-posterior dimensions over axial length L. When the first metaphyseal member is mounted on the distal femoral component with the first metaphyseal member frictionally locked to the distal femoral component, the assembly has a maximum axial length. When the second metaphyseal member is mounted on the distal femoral component with the second metaphyseal member frictionally locked to the distal femoral component, the assembly has a maximum axial length. The maximum axial length of the assembly of the second metaphyseal member and the distal femoral component is greater than the maximum axial length of the assembly of the first metaphyseal member and the distal femoral component.

Optionally, the first metaphyseal member and the second metaphyseal member have the same number of steps over axial length L of the tapered stepped outer surface of the first metaphyseal member and the tapered stepped outer surface of the second metaphyseal member.

Optionally, each step of the tapered stepped outer surface of the first metaphyseal member has an axial height and each step of the tapered stepped outer surface of the second metaphyseal member has an axial height. The axial heights of corresponding steps of the first metaphyseal member and the second metaphyseal member are the same.

Optionally, the system further comprises a third metaphyseal member including an inner surface defining a tapered bore sized and shaped to be mountable on the stem of the distal femoral component and to create a frictional lock between the stem of the distal femoral component and the first metaphyseal member. The tapered bore extends proximally from an opening at the distal end of the first metaphyseal member. The first metaphyseal member includes a tapered stepped outer surface having an axial length L+X+Y. When the third metaphyseal member is mounted on the distal femoral component with the third metaphyseal member frictionally locked to the distal femoral component, the assembly has a maximum axial length. The maximum axial length of the assembly of the third metaphyseal member and the distal femoral component is greater than the maximum axial length of the assembly of the second metaphyseal member and the distal femoral component.

Optionally, the distal femoral component has a distal bone-facing surface and the system further comprises a distal femoral augment. The distal femoral augment has a thickness that is substantially the same as X.

The invention is described below by way of example with reference to the accompanying drawings, in which:
FIG. 1 is a view of the femoral components of a modular knee prosthesis system.
FIG. 2 is a view of the tibial components of a modular knee prosthesis system.
FIG. 3 is a medial-lateral view of the smallest size of metaphyseal sleeves of the modular knee prosthesis system of FIG. 1.
FIG. 4 is a medial-lateral view of another size of metaphyseal sleeves of the modular knee prosthesis system of FIG. 1.
FIG. 5 is a medial-lateral view of another size of metaphyseal sleeves of the modular knee prosthesis system of FIG. 1.
FIG. 6 is a medial-lateral view of the largest size of metaphyseal sleeves of the modular knee prosthesis system of FIG. 1.
FIG. 7 is an anterior-posterior view of the metaphyseal sleeve of FIG. 3.
FIG. 8 is an anterior-posterior view of the metaphyseal sleeve of FIG. 4.
FIG. 9 is an anterior-posterior view of the metaphyseal sleeve of FIG. 5.
FIG. 10 is an anterior-posterior view of the metaphyseal sleeve of FIG. 6.
FIG. 11 is a cross-sectional view of the metaphyseal sleeve of FIG. 7, taken along line 11-11 of FIG. 7.
FIG. 12 is a cross-sectional view of the metaphyseal sleeve FIG. 10, taken along line 12-12 of FIG. 10.FIG. 13 is an anterior-posterior view of a modular knee prosthesis system using a standard femoral stem and the smallest size of metaphyseal sleeve.
FIG. 14 is an anterior-posterior view of a modular knee prosthesis system similar to FIG. 13 but shown with the largest size of metaphyseal sleeve.
FIG. 15 is a view of another modular prosthesis system.
FIG. 16 is a medial-lateral view of one size of metaphyseal sleeves of the modular knee prosthesis system of FIG. 15.
FIG. 17 is a medial-lateral view of another size of metaphyseal sleeves of the modular knee prosthesis system of FIG. 15.
FIG. 18 is a medial-lateral view of another size of metaphyseal sleeves of the modular knee prosthesis system of FIG. 15.
FIG. 19 is a cross sectional plan view taken along the lines 19-19 in FIGS. 16 to 18.
FIG. 20 is a cross sectional plan view taken along the lines 20-20 in FIGS. 17 and 18.
FIG. 21 is a medial-lateral view of a surgical broach of an orthopaedic surgical instrument system for use with the modular knee prosthesis system of FIG. 15.
FIG. 22 is a medial-lateral of another size of surgical broach of the orthopaedic surgical instrument system.
FIG. 23 is a medial-lateral of another size of surgical broach of the orthopaedic surgical instrument system.
FIG. 24 is a cross sectional plan view taken along the lines 24-24 in FIGS. 21 to 23.
FIG. 25 is a cross sectional plan view taken along the lines 20-20 in FIGS. 22 and 23.
FIG. 26 is a perspective view of a distal augment for use with a femoral component of the modular knee prosthesis system of FIG. 15.
FIG. 27 is a fragmentary cross sectional view of the distal augment taken along the lines 27-27 in FIG. 26.
FIG. 28 is a perspective view of another size of distal augment for use with the femoral component of FIG. 15.
FIG. 29 is a fragmentary cross sectional view of the distal augment taken along the lines 29-29 in FIG. 28.
FIG. 30 is an elevation view of the femoral component of FIG. 15 and a posterior augment.
FIG. 31 is a view similar to FIG. 30 showing the posterior augment secured to the femoral component and the distal augment of FIGS. 26 and 27.
FIG. 32 is an elevation view of the femoral component of FIG. 15 and another size of posterior augment.
FIG. 33 is a view similar to FIG. 32 showing the posterior augment secured to the femoral component and the distal augment of FIGS. 28 and 29.
FIG. 34 is a view similar to FIGS. 32 and 33 showing the installation of the distal augment of FIGS. 28 and 29.
FIG. 35 is a view similar to FIGS. 32 to 34 showing the posterior augment and the distal augment secured to the femoral component.
FIG. 36 is an anterior-posterior view of a modular knee prosthesis system of FIG. 15 using the metaphyseal sleeve of FIG. 16 and the augments of FIGS. 30 and 31.
FIG. 37 is an anterior-posterior view similar to FIG. 36 showing the metaphyseal sleeve of FIG. 18 and the augments of FIGS. 32 to 35.

Terms representing anatomical references, such as anterior, posterior, medial, lateral, superior and inferior may be used throughout this document to refer to the orthopaedic implants and orthopaedic surgical instruments described herein as well as to refer to the patient's natural anatomy. Such terms have well-understood meanings in both the study of anatomy and the field of orthopaedics. Use of such anatomical reference terms in this document is intended to be consistent with their well-understood meanings unless noted otherwise.

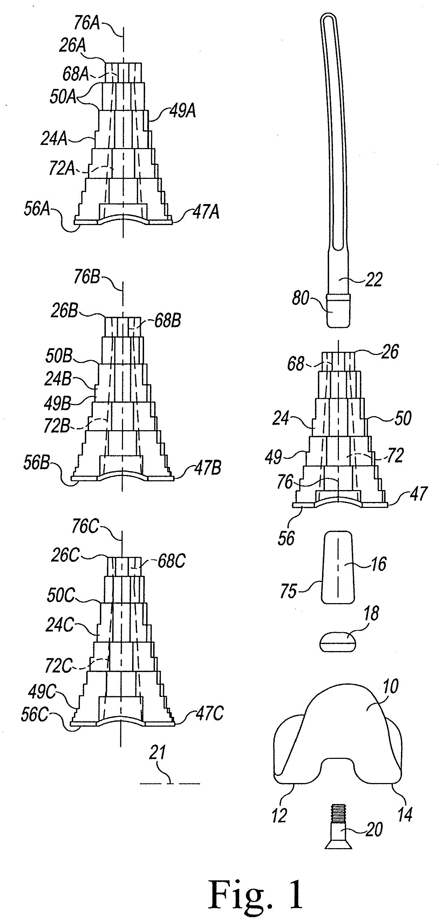

FIG. 1 shows an example of the femoral components of a modular knee prosthesis system. The femoral components of the system include a distal femoral component 10 with distal curved convex condylar surfaces 12, 14. The distal femoral component is a posterior stabilized component. The system shown in FIG. 1 also includes a femoral stem 16, along with a collar 18 for placement between the stem 16 and the distal femoral component 10 and a bolt 20 so that the stem 16 and collar 18 may be selectively mounted on the distal femoral component. Each stem 16 has a frusto-conical outer surface that is smooth and tapers from a maximum outer diameter at the distal end to smaller outer diameters at positions proximal to the distal end. Stem extensions 22 are also provided. All of the above components may be standard parts of the knee system sold under the trade mark PFC SIGMA by DePuy Synthes, for example. Each stem 16 is an adapter with features like those disclosed in US-A-2006/0030945. The stems 16 may also have features like those disclosed in US-6171342, US-5824097 and US-5782921. Also as disclosed in US-A-2006/0030945, the stem extension may have features other than those shown in FIG. 1. Optionally, the femoral component 10 may have an integral stem 16 instead of the stem adapter 16, collar 18 and bolt 20.

The femoral components of the system shown in FIG. 1 include a plurality of sizes of metaphyseal sleeves 24, 24A, 24B, 24C. As described in more detail below, the geometries of the exterior surfaces of the four sizes of metaphyseal sleeves 24, 24A, 24B, 24C are the same over a substantial portion of their axial lengths. It should be understood that multiple sizes of distal femoral components 10 and stem extensions 22 would typically be included in the modular knee prosthesis system. It should also be understood that a modular knee prosthesis system may include fewer or more sizes of metaphyseal sleeves 24, 24A, 24B, 24C.

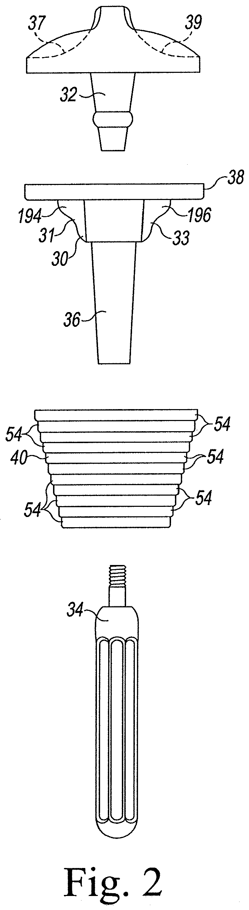

As shown in FIG. 2, on the tibial side, the kit includes a tibial tray component 30, a tibial bearing insert 32 and a stem extension 34. The tibial tray component 30 is a tibial tray as sold by DePuy Synthes under the trade mark MBT. The tray component 30 has an integral stem portion 36 with a bore (not shown) with internal threads to which the stem extension 34 may be attached. The outer surface of the stem portion 36 has a smooth finish, tapers away from the joint motion surface and is connected to the inferior surface of the tibial tray component 30 through keels 31, 33. The stem portion 36 extends distally from a platform 38, which has a proximal surface on which the tibial bearing insert 32 rests. The tibial components may also include one or more types or sizes of metaphyseal sleeves, such as sleeve 40 that has a tapered bore (not shown) sized and shaped to lock frictionally with the tapered stem portion 36 of the tibial tray component 30. Optionally, the tibial component may comprise a unitary, all-polymer component or a fixed bearing system, such as those disclosed in US-7628818 and US-8128703.

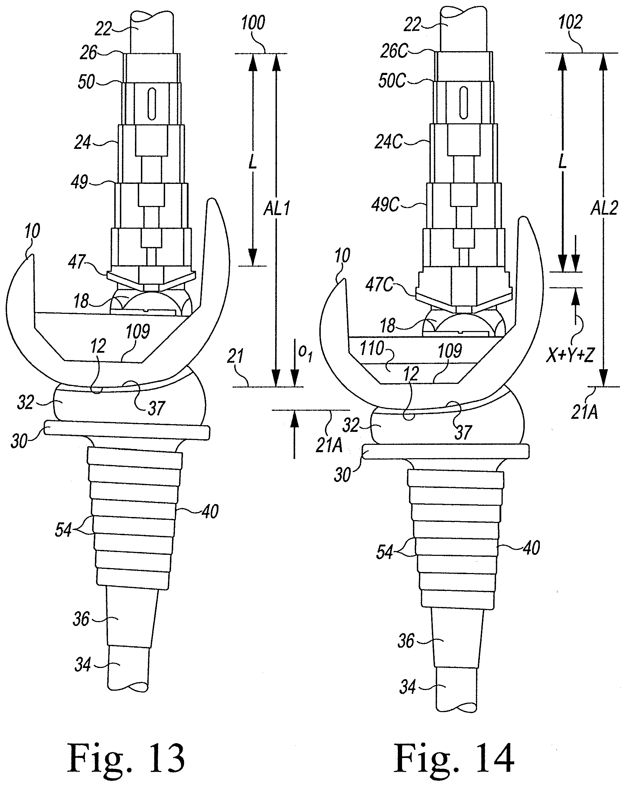

The juncture of the curved convex condyles 12, 14 of the distal femoral component 10 and the curved concave condylar surfaces of the tibial bearing insert 32 (the curved concave condylar surfaces of the tibial bearing insert being shown in FIG. 2 in phantom at 37, 39) define the articulation of the femoral and tibial components as the knee flexes and extends. When the patient's leg is in extension, the contact between the curved convex condyles 12, 14 and concave condylar surfaces 37, 39 corresponds with a distal joint line. As the knee is flexed from full extension, the distal femoral component 10 and tibial bearing insert 32 move with respect to each other so that the joint line at full flexion (when the posterior surfaces of the femoral condyles contact the bearing surface) may vary somewhat from the distal joint line. The plane of the joint line, tangent to the point of contact of the condylar surfaces of the distal femoral component on the tibial insert, is shown at 21 in FIGS. 1 and 13 and at 21A in FIG. 14.

It should be understood that a typical modular knee prosthesis system or kit would include multiple sizes of each of the tibial components 30, 32, 34, 40.

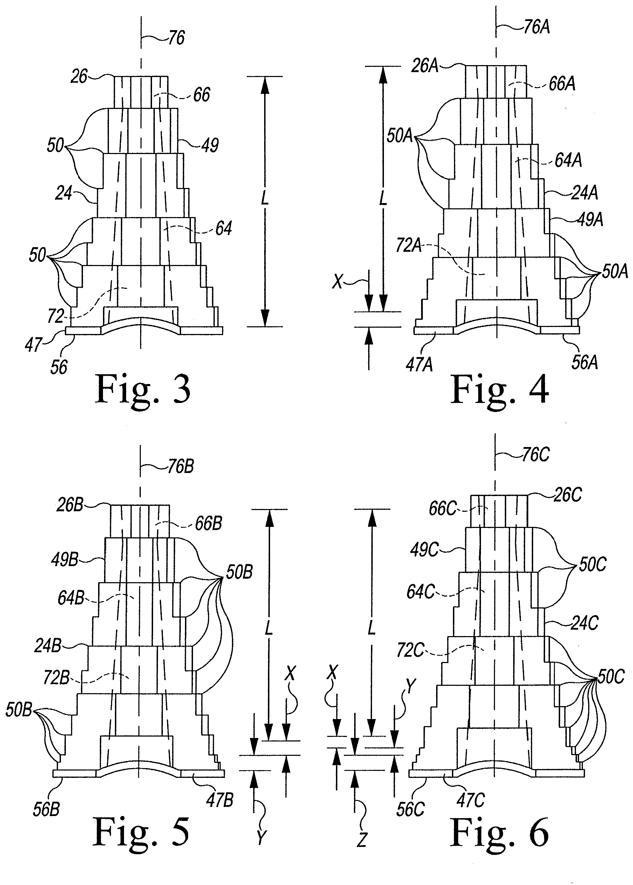

The metaphyseal sleeves 24, 24A, 24B, 24C are designed for use in a bone in which the condition of the bone requires additional support or fixation in the metaphysis of the bone. Each of the femoral sleeves 24, 24A, 24B, 24C has an outer surface that includes a distal base 47, 47A, 47B, 47C and a stepped portion 49, 49A, 49B, 49C extending proximally from the distal base to the proximal ends 26, 26A, 26B, 26C. Each stepped portion 49, 49A, 49B, 49C has a plurality of adjacent steps or terraces, shown in FIGS. 3-10 at 50, 50A, 50B and 50C for the femoral sleeves 24, 24A, 24B, 24C and at 54 for the tibial sleeve 40 (FIG. 2). For the femoral sleeves, the stepped outer surfaces taper proximally: the steps 50, 50A, 50B, 50C at the distal ends 56, 56A, 56B, 56C have the largest anterior-posterior and medial-lateral dimensions and the steps 50, 50A, 50B, 50C at the proximal ends 26, 26A, 26B, 26C have the smallest anterior-posterior and medial-lateral dimensions; the intermediate steps gradually become smaller from the distal ends 56, 56A, 56B, 56C toward the proximal ends 26, 26A, 26B, 26C. For the tibial sleeve 40, the outer surface tapers distally: the most distal step has the smallest anterior-posterior and medial-lateral dimensions and the most proximal step has the largest anterior-posterior and medial-lateral dimensions; the intermediate steps gradually become smaller from the proximal end toward the distal end.

It should be understood that the number and size of the steps 50, 50A, 50B, 50C, may vary. For example, the outer surfaces of the metaphyseal sleeves 24, 24A, 24B, 24C, may have steps and be shaped like standard commercially available metaphyseal sleeves sold by DePuy Synthes, and may be configured like known sleeves, for example as disclosed in US-7799085. The outer surfaces of the sleeves 24, 24A, 24B, 24C, may also be porous coated to promote bone ingrowth, as in known products. The porous coating may extend over substantially all or a portion of the stepped outer surfaces of the sleeves 24, 24A, 24B, 24C.

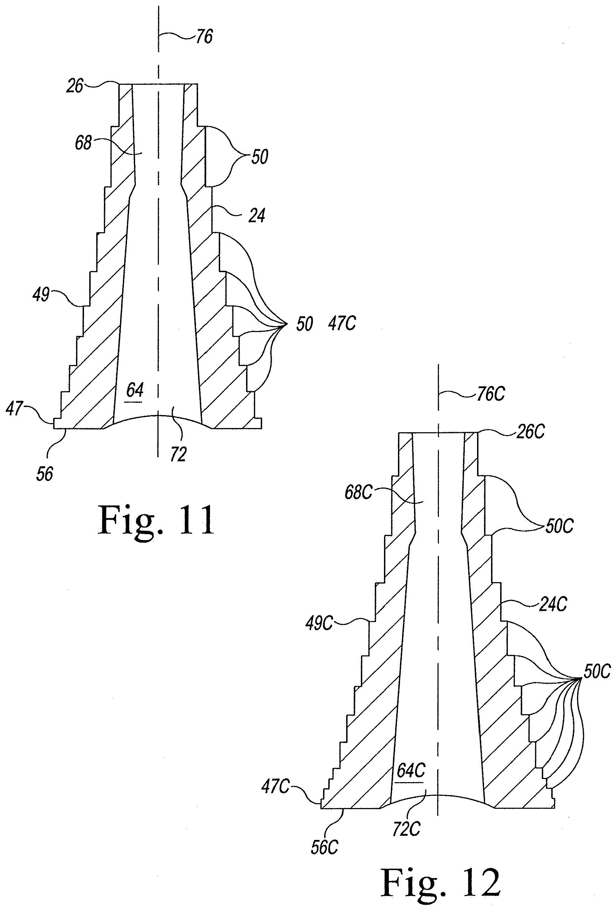

As shown in FIGS. 1, 3 to 6, 11 and 12, the femoral sleeves 24, 24A, 24B, 24C have interior surfaces 64, 64A, 64B, 64C defining a proximal bore 68, 68A, 68B, 68C and a distal bore 72, 72A, 72B, 72C. The proximal and distal bores 68, 68A, 68B, 68C, 72, 72A, 72B, 72C in each femoral sleeve may be connected and aligned along central longitudinal axes 76, 76A, 76B, 76C of the bores.

The proximal bores 68, 68A, 68B, 68C of the femoral sleeves 24, 24A, 24B, 24C are sized and shaped to receive a distal end 80 of a stem extension 22. Accordingly, for a stem extension having a Morse taper post at its distal end, the proximal bore would comprise a Morse taper bore sized and shaped to receive and frictionally lock with the Morse taper post. Alternatively, for a stem extension having a threaded distal end, the proximal bore may be threaded to receive and lock to the threaded distal end of the stem extension. An adapter to allow for use of different types of stem extensions may also be used, for example as disclosed in US-7799085.

The distal bores 72, 72A, 72B, 72C of the femoral metaphyseal sleeves 24, 24A, 24B, 24C are frusto-conical Morse taper bores, tapering from the distal ends 56, 56A, 56B, 56C of the sleeves 24, 24A, 24B, 24C toward the proximal ends 26, 26A, 26B, 26C of the sleeves 24, 24A, 24B, 24C. These distal bores 72, 72A, 72B, 72C are sized, shaped and finished to be mountable on the stem or adapter 16 of the distal femoral component 10 and to create a frictional lock between the stem of the distal femoral component and the metaphyseal sleeve, the stem or adapter 16 defining a Morse taper post.

As used herein, "Morse taper" refers to one type of locking tapers between mating components. Generally, Morse taper posts and bores have frusto-conical shapes, substantially the same taper angle and have complementary outer and inner diameters at some point along their length to allow for tight frictional engagement between the posts and the walls defining the bores. Standard taper angles and standard surface finishes for such locking tapers may be used in the present invention. It should be appreciated that other types of tapered components may be used.

The distal bores 72, 72A, 72B, 72C of each size of sleeve 24, 24A, 24B, 24C has the same maximum inner diameter at the distal end 56, 56A, 56B, 56C of the sleeve. This maximum inner diameter substantially corresponds with the maximum outer diameter of the tapered frusto-conical outer surface 75 of the stem or adapter 16 of the distal femoral component 10. The distal bores 72, 72A, 72B, 72C of all the sizes of sleeves 24, 24A, 24B, 24C and the tapered frusto-conical outer surface 75 of the stem or adapter 16 taper in the proximal direction at substantially the same taper angle so that relative axial movement of the sleeve 24, 24A, 24B, 24C and stem or adapter 16 locks the two together when the interior surface 64, 64A, 64B, 64C of the sleeve 24 engages and frictionally locks with the tapered frusto-conical outer surface 75 of the stem or adapter 16.

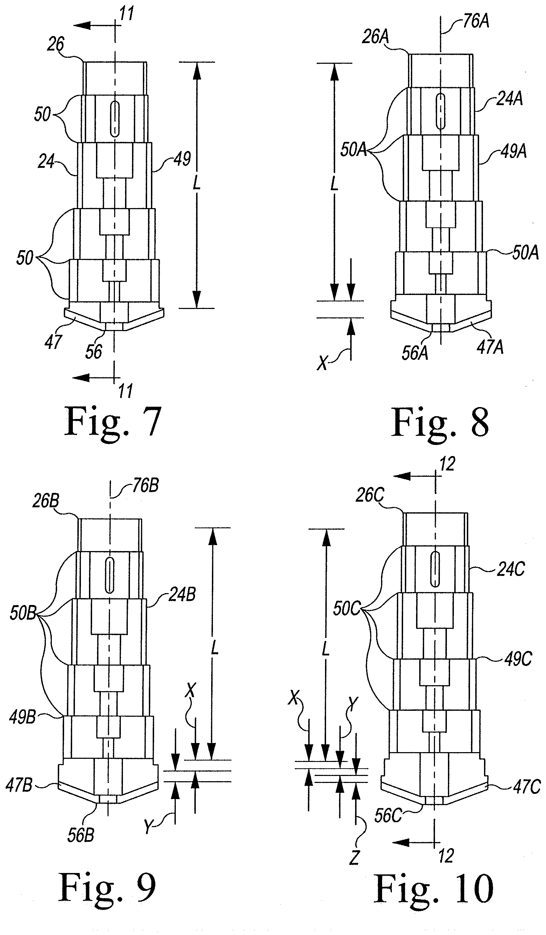

As shown in FIGS. 3, 7 and 13, the stepped outer surface 49 of the smallest size of femoral metaphyseal sleeve 24 has an overall axial length between the distal base 47 and the proximal end 26 shown at "L". The stepped outer surface 49A of the next larger size of femoral metaphyseal sleeve 24A has an overall axial length between the base 47A and the proximal end 26A of "L+X", the dimensions "L" and "X" being shown in FIGS. 4 and 8. The stepped outer surface 49B of the next larger size of femoral metaphyseal sleeve 24B has an overall axial length between the base 47B and the proximal end 26B of "L+X+Y", the dimensions "L", "X" and "Y" being shown in FIGS. 5 and 9. The stepped outer surface 49C of the largest size of femoral metaphyseal sleeve 24C has an overall axial length between the base 47C and the proximal end 26C of "L+X+Y+Z", the dimensions "L", "X", "Y" and "Z" being shown in FIGS. 6, 10 and 14. The different sizes of femoral metaphyseal sleeves may be provided with differences of a few millimetres (for example, 4 mm) between each size, so that X=4 mm, Y=4 mm and Z=4 mm. It should be understood that these dimensions are provided as examples only.

The geometries of the stepped outer surfaces 49, 49A, 49B, 49C of all sizes of femoral metaphyseal sleeve 24, 24A, 24B, 24C are essentially identical over axial length "L". Thus, if "L" is 68 mm for the smallest sleeve, the sizes and shapes of the proximal 68 mm of the other sleeve sizes 24A, 24B, 24C are essentially identical to the size and shape of the proximal 68 mm of the smallest sleeve 24. In other words, over axial length "L" for all of the sizes of femoral metaphyseal sleeves 24, 24A, 24B, 24C, the sleeves have the same number of steps, and each step has the same maximum medial-lateral dimension, the same maximum anterior-posterior dimension, the same axial height and the same shape. The different sizes of femoral metaphyseal sleeves differ only in the sizes of the bases 47, 47A, 47B, 47C and in the distal portions corresponding with the axial extensions of the sleeves beyond the length "L" of the smallest sleeve 24.

| Femoral sleeve | Maximum A-P dimension at "L" | Maximum A-P dimension distal to "L" | Maximum M-L dimension at "L" | Maximum M-L dimension distal to "L" |
|---|---|---|---|---|
| 24 | 22 mm | Not applicable | 34 mm | Not applicable |
| 24A | 22 mm | 24 mm | 34 mm | 40 mm |
| 24B | 22 mm | 24 mm | 34 mm | 46 mm |
| 24C | 22 mm | 26 mm | 34 mm | 52 mm |

FIGS. 13 and 14 show assemblies of the smallest and largest femoral metaphyseal sleeves 24, 24C with a distal femoral implant component 10, femoral stem extension 22, tibial tray 30, tibial insert 32 and tibial stem extension 34. The assemblies have maximum axial lengths from planes at the proximal ends 26, 26C (the planes shown at 100 and 102) to the plane of the joint line, shown at 21 in FIG. 13 and at 21A in FIG. 14. These maximum axial lengths of the assemblies are shown at AL1 in FIG. 13 and AL2 in FIG. 14. AL2 is longer than AL1 by the dimension "X+Y+Z", that is the axial length of the sleeve 24C beyond the length "L" of the smallest sleeve 24.

As can also be seen from a comparison of FIGS. 13 and 14, using the larger sleeve 24C distalizes the joint line 21 to the position 21A by the offset distance o₁. This offset distance o₁ also corresponds with the dimension "X+Y+Z". Similarly, using the sleeve 24A distalizes the joint line by the dimension "X" and using the sleeve 24B distalizes the joint line by the dimension "X+Y".

Since the geometries of the stepped outer surfaces 49, 49A, 49B, 49C of the different sizes of sleeves 24, 24A, 24B, 24C are the same through axial length "L", the surgeon can prepare the distal femur to receive the smallest size of femoral sleeve 24. If the surgeon determines intraoperatively that the joint line should be distalized, the surgeon may use any of the other sizes of sleeve 24A, 24B, 24C, and the proximal portion of the larger size sleeve will fit within the opening prepared in the femur to receive the smaller sleeve and extend distally from the bone by the distance "X", "X+Y" or "X+Y+Z" thereby to offset the joint line distally. The surgeon can accomplish this distalization without any further preparation of the bone cavity.

As described above, femoral augments may be used on the distal and posterior bone-facing surfaces of the femoral implant components when the joint line is distalized. In the device shown in the drawings, a distal augment 110 may be attached to one of a pair of distal fixation surfaces 109 of the distal femoral implant component 10 when the sleeve 24C is used, as shown in FIG. 14. In the device shown in the drawing, the thickness of the augment 109 is equal to the offset distance o₁. One of the distal fixation surfaces 109 is positioned opposite the condylar surface 12 and the other distal fixation surface 109 is positioned opposite the condylar surface 14. It should be appreciated that another distal augment may be attached to that surface as well. When the sleeve 24 is used, no augment is necessary, as shown in FIG. 13.

The design features discussed above may also be applied to the tibial components of the knee implant system, such as the tibial sleeve 40 shown in FIG. 2. Such a system could allow the surgeon to select components to provide a proximal offset to the tibial tray platform 38.

All of the components of the prosthesis systems described herein may be made of standard materials, such as a standard polymer (UHMWPE, for example) for the tibial bearing insert 32 and standard metals, such as cobalt-chromium and titanium alloys, for the remaining components. To promote bone ingrowth, the sleeves 24, 24A, 24B, 24C may be porous coated, or could comprise titanium foam as disclosed in US-A-2010/0057212 and US-A-2010/0076565.

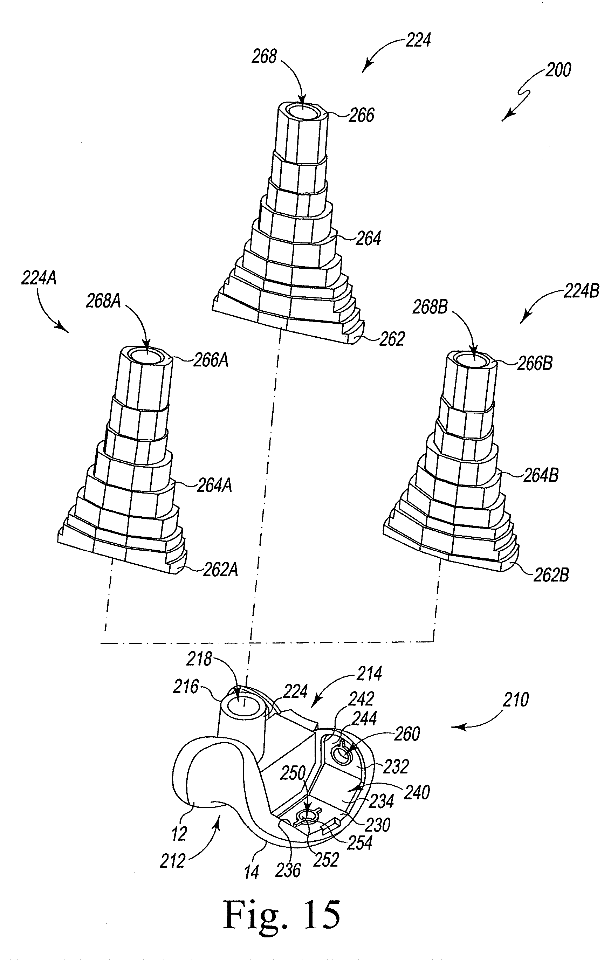

Referring now to FIGS. 15 to 37, another modular knee prosthesis system is shown with different femoral components (hereinafter components 200). An orthopaedic surgical instrument system 300 for use with the modular knee prosthesis system is also shown. Some features of the devices shown in FIGS. 15 to 37 are substantially similar to those discussed above with reference to the device shown in FIGS. 1 to 14. Such features are designated in FIGS. 15 to 37 with the same reference numbers as those used in FIGS. 1 to 14.

As shown in FIG. 15, the femoral components 200 of the system include a femoral component 210 including distal curved convex condylar surfaces 12, 14. The surfaces 12, 14 are shaped (i.e., curved) in a manner that approximates the condyles of the natural femur. In the device shown in the drawings, the condylar surface 12 is a medial condyle surface 12, and the condylar surface 14 is a lateral condyle surface 14. The surfaces 12, 14 are spaced apart from one another, thereby defining an intercondylar notch between them.

The condyle surfaces 12, 14 are formed in a bearing surface 212 of the femoral component 210, and the femoral component 210 includes a fixation surface 214 positioned opposite the bearing surface 212. The femoral component 210 also includes an elongated stem post 216 that extends superiorly away from the surface 214. The elongated stem post 216 is configured to receive a stem component such as, for example, the stem extension 22, or engage a metaphyseal sleeve such as, for example, the sleeves 24, 24A, 24B, 24C described above or sleeves 224, 224A, 224B, which are described in greater below.

Specifically, as shown in FIG. 15, the elongated stem post 216 of the femoral component 210 has a tapered bore 218 defined therein into which the tapered distal end 80 of the stem extension 22 may be advanced to taper lock the post 216 to the stem extension 22. Similar to the adaptor 16 of the distal femoral component 10, the outer surface 220 of the elongated stem post 216 is also tapered and may be advanced into one of the distal bores 72, 72A, 72B, 72C of the sleeves 24, 24A, 24B, 24C, respectively, or one of the tapered distal bores 272, 272A, 272B of the sleeves 224, 224A, 224B. As described above, each distal bore is shaped and finished to create a frictional lock such as, for example, a taper lock, between the corresponding sleeve and the femoral component 210. In the device shown in the drawing, the outer surface 220 defines a Morse taper.

The fixation surface 214 of the femoral component 210 includes a number of surfaces 230, 232, 234, 236 positioned opposite the condyle surfaces 12, 14. The fixation surface 214 includes a pair of distal fixation surfaces 230 similar to the distal fixation surface 109 of distal femoral component 10. One of the distal fixation surfaces 230 is positioned medially and the other is positioned laterally. The fixation surface 214 also includes a pair of posterior fixation surfaces 232, with one being medially positioned and the other laterally positioned. As shown in FIG. 15, the posterior fixation surfaces 232 extend generally in the superior/inferior direction.

The fixation surface 214 also includes a pair of posterior chamfer surfaces 234, with one being medially positioned and the other laterally positioned. The medial and lateral posterior-chamfer fixation surfaces 234 extend superiorly and posteriorly from their respective lateral and medial distal fixation surfaces 230 to their respective posterior fixation surfaces 232. As shown in FIG. 15, the fixation surface 214 has a pair of anterior chamfer surfaces 236, with one being medially positioned and the other laterally positioned. The medial and lateral anterior-chamfer fixation surfaces 236 extend superiorly and posteriorly from their respective lateral and medial distal fixation surfaces 230 to their respective posterior fixation surfaces 232.

Each of the fixation surfaces 230, 232, 234, 236 has a cement pocket formed therein. In the device shown in the drawings, the cement pockets are contiguous with one another such that a single, continuous cement pocket 240 is formed in both the medial and lateral fixation surfaces 214 of the femoral component 210. Each cement pocket 240 is defined by a side wall 242 that extends inwardly from the respective fixation surface 214 to a bottom wall 244. A mounting aperture 250 is defined in each distal fixation surface 230. As shown in FIG. 15, the aperture 250 is defined by a cylindrical wall 252 that extends inwardly from a rim 254 positioned in the cement pocket 240. As described in greater detail below, the aperture 250 is sized to receive a mounting plug 256 of a distal augment component 342, 344 to secure the augment component 342, 344 to the femoral component 210.

Another mounting aperture 260 is defined in each posterior fixation surface 232. As shown in FIG. 15, the aperture 260 is defined by a cylindrical wall 252 that extends inwardly from a rim 254 positioned in the cement pocket 240. As described in greater detail below, the aperture 260 is sized to receive a mounting plug 256 of a posterior augment component 346, 348 to secure the augment component 346, 348 to the femoral component 210. As shown in FIG. 15, the femoral components 200 include a plurality of sizes of metaphyseal sleeves 224, 224A, 224B. Similar to the sleeves 24, 24A, 24B, 24C described above, the geometries of the sleeves 224, 224A, 224B of the exterior surfaces of the three sizes are the same over a portion of their axial lengths. As used herein, the terms "same", "match", and "identical" refer to components that are designed to have the same dimensions and configuration. Such components may be subject to accepted tolerances or manufacturing variations that cause the components to vary slightly in some respect. For example, the portions of metaphyseal sleeves 224, 224A, 224B that are designed to be the same may nevertheless vary slightly due to manufacturing tolerances. Nevertheless, such components are the same, match, or are identical because they are designed to have the same configuration and dimensions. It should be understood that multiple sizes of femoral components 210 would typically be included in the modular knee prosthesis system. A modular knee prosthesis system may include fewer or more sizes of metaphyseal sleeves 224, 224A, 224B.

Similar to the sleeves 24, 24A, 24B, 24C, the sleeves 224, 224A, 224B are designed for use with bones in which the condition of the bone requires additional support or fixation in the metaphysis of the bone. As shown in FIG. 15, each of the sleeves 224, 224A, 224B has a distal base 262, 262A, 262B and a body 264, 264A, 264B extending proximally from its respective distal base to a respective proximal end 266, 266A, 266B.

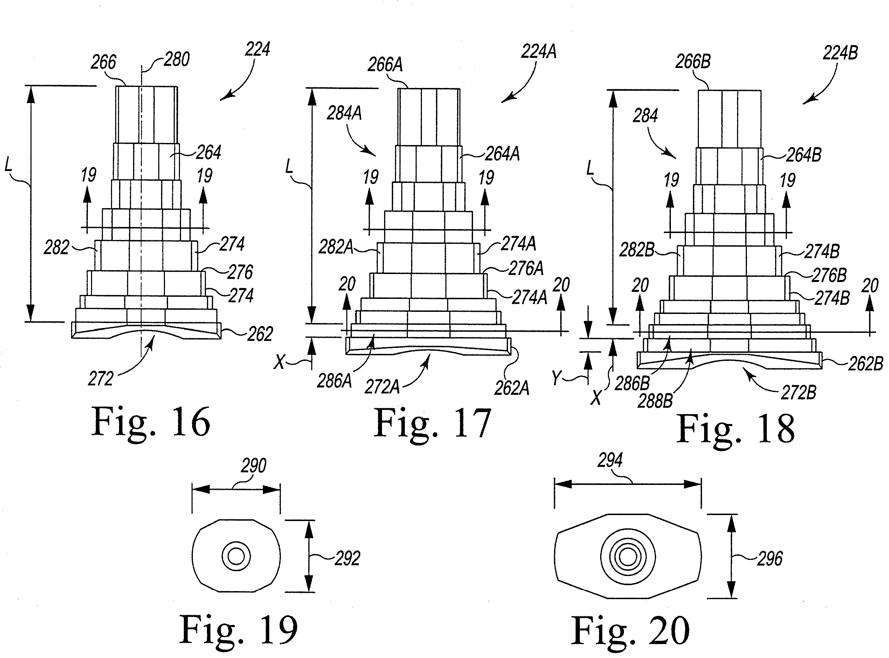

As shown in FIG. 15, each of the sleeves 224, 224A, 224B has a proximal bore 268, 268A, 268B defined in the proximal end 266, 266A, 266B thereof. The proximal bores 268, 268A, 268B, of the femoral sleeves 224, 224A, 224B are sized and shaped to receive a distal end 80 of a stem extension 22. Accordingly, for a stem extension having a tapered post at its distal end, the proximal bore would comprise a tapered bore sized and shaped to receive and frictionally lock with the tapered post. Alternatively, for a stem extension having a threaded distal end, the proximal bore may be threaded to receive and lock to the threaded distal end of the stem extension. As described above, each of the sleeves 224, 224A, 224B has a distal bore 272, 272A, 272B, which is defined in the respective distal base 262, 262A, 262B of each sleeve, as shown in FIGS. 16 to 18.

Referring now to FIGS. 16 to 18, the bodies 264, 264A, 264B of the sleeves 224, 224A, 224B include a plurality of stepped walls 274, 274A, 274B.

Each pair of adjacent stepped walls 274, 274A, 274B is connected by an annular surface 276, 276A, 276B. As a result, the bodies 264, 264A, 264B are terraced similar to the sleeves 24, 24A, 24B, 24C. In the device shown in the drawings, the bodies 264, 264A, 264B are tapered such that the sleeves 224, 224A, 224B have the smallest anterior-posterior dimensions and the smallest medial-lateral dimensions at their respective proximal ends 266, 266A, 266B and become progressively larger as the bodies 264, 264A, 264B extend to their respective distal bases 262, 262A, 262B.

It should be understood that the number and size of the stepped walls 274, 274A, 274B may vary. The outer surfaces of the sleeves 262, 262A, 262B may also be porous coated to promote bone ingrowth, as disclosed in the prior art; the porous coating may extend over substantially all or a portion of the stepped outer surfaces of the sleeves 224, 224A, 224B. It should be understood that these dimensions are provided as examples only and may vary. As shown in FIG. 16, the body 264 of the sleeve 224 has a longitudinal axis 280 and a tapered outer surface 282 that has an axial length "L" defined along the axis 280. In the device shown in the drawing, the annular surfaces 276 are substantially flat. As a result, the stepped walls 274 combine to define the axial length L of the body 264. In the device shown in the drawings, the axial length L is equal to approximately 45 mm.

As shown in FIG. 17, the body 264A of the sleeve 224A has a longitudinal axis 280A and a tapered outer surface 282A. The tapered outer surface 282A has a proximal section 284A that has an axial length "L" defined along the axis 280A and a section 286A extending distally from the proximal section 284A to the distal base 262A. The section 286A has an axial length "X" defined along the axis 280A. As a result, the overall axial length of the body 264A is "L+X". In the device shown in the drawing, the axial length "L+X" is equal to approximately 50 mm.

As shown in FIG. 18, the body 264B of the sleeve 224B has a longitudinal axis 280B and a tapered outer surface 282B. The tapered outer surface 282B has a proximal section 284B that has an axial length "L" defined along the axis 280B and a section 286B extending distally from the proximal section 284A. The section 286A has an axial length "X" defined along the axis 280B. The tapered outer surface 282B has another section 288B that extends distally from the section 286B to the distal base 262B. The section 288B has an axial length "Y" defined along the axis 280B. As a result, the overall axial length of the body 264A is "L+X+Y". In the device shown in the drawing, the axial length "L+X+Y" is equal to approximately 55 mm.

In the device shown in the drawing, the different sizes of femoral metaphyseal sleeves may be provided with differences of a few millimetres (for example, 5 millimetres) between each size, so that X=5 mm and Y=5 mm. Additionally, the overall axial length of the different sleeves may vary.

For example, the overall axial lengths of the sleeves may be between 30 mm and 55 mm.

Optionally, the outer geometries of the tapered outer surfaces 282, 282A, 282B of all sizes of femoral metaphyseal sleeve 224, 224A, 224B are essentially identical over axial length "L". Thus, if "L" of the sleeve 224 is 45 mm, the sizes and shapes of the proximal 45 mm of the other sleeve sizes 224A, 224B (i.e., the proximal sections 284A, 284B) are essentially identical to the size and shape of the proximal 45 mm of the sleeve 224. In other words, over axial length "L" for all of the sizes of femoral metaphyseal sleeves 224, 224A, 224B, the sleeves have the same number of stepped walls, as shown in FIGS. 16 to 18. Further, as shown in FIG. 19, each stepped wall over axial length "L" also has the same maximum medial-lateral dimension 290, the same maximum anterior-posterior dimension 292, and the same shape. Each stepped wall over axial length "L" also has the same axial height, as shown in FIGS. 16 to 18.

The different sizes of femoral metaphyseal sleeves 224, 224A, 224B differ only in the sizes of the bases 262, 262A, 262B and in the distal portions corresponding with the axial extensions of the sleeves beyond a given axial length. For example, the tapered outer surfaces 282A, 282B of the femoral metaphyseal sleeve 224A, 224B are essentially identical over axial length "X". Thus, if "X" of the sleeve 224A is 5 mm, the size and shape of the section 286B of the sleeve 224B is essentially identical to the size and shape of the section 286A of the sleeve 224A. In other words, over axial length "X" for the sizes of femoral metaphyseal sleeves 224A, 224B, the sleeves have the same number of stepped walls, as shown in FIGS. 17 to 18. Further, as shown in FIG. 20, each stepped wall has the same maximum medial-lateral dimension 294, the same maximum anterior-posterior dimension 296, and the same shape. Nevertheless, the femoral metaphyseal sleeve 224B differs from the sleeve 224A in the size of the base 262B and in the configuration of the distal section 288B.

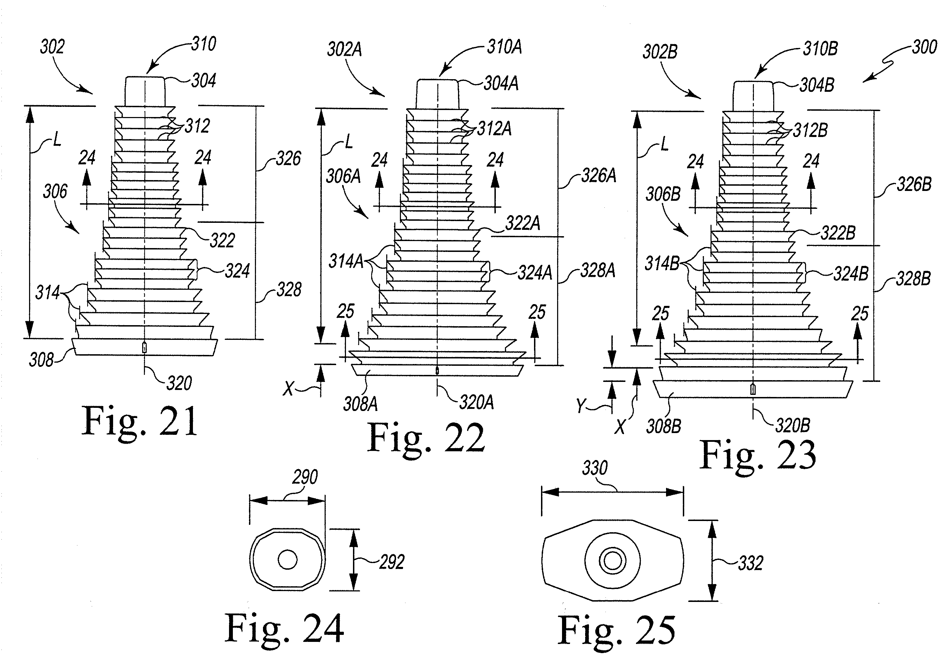

FIGS. 21 to 25 show a plurality of surgical instruments 300, which may be used with the femoral components 200. The surgical instruments 300 are a plurality of sizes of surgical broaches 302, 302A, 302B. Each of the broaches 302, 302A, 302B is formed from a metallic material such as, for example, stainless steel or cobalt chromium. As described in greater detail below, the outer geometries of the broaches 302, 302A, 302B are the same over a portion of their axial lengths and correspond to the outer geometries of the metaphyseal sleeves 224, 224A, 224B. As described above, the femoral components 200 may include fewer or more sizes of metaphyseal sleeves 224, 224A, 224B; it should be appreciated that the surgical instruments 300 may include fewer or more sizes of broaches 302, 302A, 302B.

Each of the broaches 302, 302A, 302B includes a proximal tip 304, 304A, 304B and a body 306, 306A, 306B extending from the proximal tip 304, 304A, 304B to a respective distal end 308, 308A, 308B. The tip 304, 304A, 304B of each broach 302, 302A, 302B has an aperture 310, 310A, 310B defined therein that is sized to receive a femoral stem trial. The distal end 308, 308A, 308B of each broach 302, 302A, 302B is configured to engage an attachment mechanism of an instrument handle.

The bodies 306, 306A, 306B have a plurality of cutting teeth 312, 312A, 312B defined in the outer surface 322, 322A, 322B thereof. The cutting teeth 312, 312A, 312B are configured to engage the bone surrounding the medullary canal of the patient's femur to define a cavity in the bone sized to receive a sleeve. The cutting teeth 312, 312A, 312B cooperate to define a plurality of stepped planes 314, 314A, 314B of their respective outer surfaces 322, 322A, 322B. As a result, the bodies 306, 306A, 306B are terraced. The bodies 306, 306A, 306B are tapered such that the broaches 302, 302A, 302B have the smallest anterior-posterior dimensions and the smallest medial-lateral dimensions at their respective proximal tips 304, 304A, 304B and become progressively larger as the bodies 306, 306A, 306B extend to their respective distal ends 308, 308A, 308B. The number of stepped planes 314, 314A, 314B of the broaches 302, 302A, 302B corresponds to the number of the stepped walls 274, 274A, 274B of the sleeves 224, 224A, 224B, respectively.

As shown in FIG. 21, the body 306 of the broach 302 has a longitudinal axis 320 and a tapered outer surface 322 defined by the tips 324 of the cutting teeth 312. The tapered outer surface 322 has an axial length "L" defined along the axis 320. In that way, the body 306 has the same axial height as the body 264 of the sleeve 224. Additionally, the stepped planes 314 of the body 306 combine to define the axial length L of the body 306. The number of stepped planes 314 is equal to the number of stepped walls 274 of the sleeve 224; as such, each stepped plane 314 corresponds to a stepped wall 274 of the sleeve 224.

The tapered outer surface 322 of the broach 302 has a proximal section 326 extending from the proximal tip 304 and a section 328 extending from the proximal section 326 to the distal end 308. The distal section 328 has an axial length that is approximately 50% of the axial length L.

In the proximal section 326 of the tapered outer surface 322, the outer geometry of the broach 302 defined by the stepped planes 314 is the same as the corresponding outer geometry of the sleeve 224 defined by the stepped walls 274. In other words, the number of stepped planes 314 is equal to the number of stepped walls 274, and each stepped plane 314 has the same maximum medial-lateral dimension, the same maximum anterior-posterior dimension, and the same axial height as the corresponding stepped wall 274. For example, as shown in FIG. 24, the stepped planes 314 in the proximal section 326 define the same maximum medial-lateral dimension 290 and the same maximum anterior-posterior dimension 292 as the corresponding stepped wall 274 of the sleeve 224. As a result, the broach 302 is configured to define a cavity in the patient's femur that includes a proximal section that is substantially the same as the sleeve 224 such that the sleeve 224 is fitted into that section.

In the distal section 328 of the tapered outer surface 322 the number of stepped planes 314 is equal to the number of stepped walls 274, and each stepped plane 314 has the same axial height as the corresponding stepped wall 274. However, the maximum medial-lateral dimension and the maximum anterior-posterior dimension of each stepped plane 314 are smaller than the maximum medial-lateral dimension and the maximum anterior-posterior dimension of the corresponding stepped wall 274. In other words, the outer geometry of the broach 302 defined by the stepped planes 314 is smaller than the corresponding outer geometry of the sleeve 224 defined by the stepped walls 274. As a result, the broach 302 is configured to define a cavity in the patient's femur that includes a distal section that is smaller than the sleeve 224 such that the sleeve 224 is press fit into that section.

The maximum medial-lateral dimension of each stepped plane 314 in the distal section 328 is 0.35 mm less than the maximum medial-lateral dimension of the corresponding stepped wall 274 of the sleeve 224. Similarly, the maximum anterior-posterior dimension of each stepped plane 314 in the distal section 328 is 0.35 mm less than the maximum anterior-posterior dimension of the corresponding stepped wall 274 of the sleeve 224. It should be appreciated that the dimensions of the broach 302 may be adjusted to provide greater or less press fit for the sleeve 224.

Furthermore, since the geometries of the outer surfaces 282, 282A, 282B of the femoral metaphyseal sleeves 224, 224A, 224B are essentially identical through axial length "L", the sleeves 224A, 224B will fit within a cavity prepared by the broach 302 in a patient's femur and extend distally from the bone by the distance "X" or "X+Y". As such, if the sleeve 224A is inserted into a cavity prepared by the broach 302, the portion of the sleeve 224 corresponding to the distal section 328 of the broach 302 will be press fit, while the portion corresponding to the proximal section 326 of the broach 302 will be fitted into that portion of the cavity.

As shown in FIG. 22, the body 306A of the broach 302A has a longitudinal axis 320A and a tapered outer surface 322A defined by the tips 324A of the cutting teeth 312A. The tapered outer surface 322A has an axial length "L+X" defined along the axis 320A. In that way, the body 306A has the same axial height as the sleeve 224A. Additionally, the stepped planes 314A of the body 306A combine to define the axial length "L+X" of the body 306A. The number of stepped planes 314A is equal to the number of stepped walls 274A of the sleeve 224A; as such, each stepped plane 314A corresponds to a stepped wall 274A of the sleeve 224A.

The tapered outer surface 322A of the broach 302A has a proximal section 326A extending from the proximal tip 304A and a section 328A extending from the proximal section 326A to the distal end 308A. The distal section 328A has an axial length that is approximately 50% of the axial length "L+X".

In the proximal section 326A of the tapered outer surface 322A, the outer geometry of the broach 302A defined by the stepped planes 314A is the same as the corresponding outer geometry of the sleeve 224A defined by the stepped walls 274A. In other words, the number of stepped planes 314A is equal to the number of stepped walls 274A, and each stepped plane 314A has the same maximum medial-lateral dimension, the same maximum anterior-posterior dimension, and the same axial height as the corresponding stepped wall 274A. For example, as shown in FIG. 24, the stepped planes 314A in the proximal section 326A define the same maximum medial-lateral dimension 290 and the same maximum anterior-posterior dimension 292 as the corresponding stepped wall 274A of the sleeve 224A. As a result, the broach 302A is configured to define a cavity in the patient's femur that includes a proximal section that is substantially the same as the sleeve 224A such that the sleeve 224A is fitted into that section.

In the distal section 328A of the tapered outer surface 322A the number of stepped planes 314A is equal to the number of stepped walls 274A, and each stepped plane 314A has the same axial height as the corresponding stepped wall 274A. However, the maximum medial-lateral dimension and the maximum anterior-posterior dimension of each stepped plane 314A are smaller than the maximum medial-lateral dimension and the maximum anterior-posterior dimension of the corresponding stepped wall 274A. In other words, the outer geometry of the broach 302A defined by the stepped planes 314A is smaller than the corresponding outer geometry of the sleeve 224A defined by the stepped walls 274A.

For example, as shown in FIG. 25, a stepped plane 314A in the distal section 328A defines a maximum medial-lateral dimension 330 that is less than the maximum medial-lateral dimension 294 of the corresponding stepped wall 274A. Similarly, the same stepped plane 314A in the distal section 328A defines a maximum anterior-posterior dimension 332 that is less than the maximum medial-lateral dimension 294 of the corresponding stepped wall 274A. As a result, the broach 302A is configured to define a cavity in the patient's femur that includes a distal section that is smaller than the sleeve 224A such that the sleeve 224A is press fit into that section.

The maximum medial-lateral dimension of each stepped plane 314A in the distal section 328A is 0.35 mm less than the maximum medial-lateral dimension of the corresponding stepped wall 274A of the sleeve 224A. Similarly, the maximum anterior-posterior dimension of each stepped plane 314A in the distal section 328A is 0.35 mm less than the maximum anterior-posterior dimension of the corresponding stepped wall 274A of the sleeve 224A. It should be appreciated that the dimensions of the broach 302A may be adjusted to provide greater or less press fit for the sleeve 224A. It should also be appreciated that the medial-lateral press fit and the anterior-posterior press fit may or may not be equal.

Furthermore, since the geometries of the outer surfaces 282A, 282B of the femoral metaphyseal sleeves 224A, 224B are essentially identical through axial length "L+X", the sleeve 224B will fit within a cavity prepared by the broach 302A in a patient's femur and extend distally from the bone by the distance "X+Y". As such, if the sleeve 224B is inserted into a cavity prepared by the broach 302A, the portion of the sleeve 224B corresponding to the distal section 328A of the broach 302A will be press fit, while the portion of the sleeve 224B corresponding to the proximal section 326A of the broach 302A will be fitted into that portion of the cavity.

As shown in FIG. 23, the body 306B of the broach 302B has a longitudinal axis 320B and a tapered outer surface 322B defined by the tips 324B of the cutting teeth 312B. The tapered outer surface 322B has an axial length "L+X+Y" defined along the axis 320B. In that way, the body 306B has the same axial height as the sleeve 224B. Additionally, the stepped planes 314B of the body 306B combine to define the axial length "L+X+Y" of the body 306B. The number of stepped planes 314B is equal to the number of stepped walls 274B of the sleeve 224B; as such, each stepped plane 314B corresponds to a stepped wall 274B of the sleeve 224B.

The tapered outer surface 322B of the broach 302B has a proximal section 326B extending from the proximal tip 304B and a section 328B extending from the proximal section 326B to the distal end 308B. The distal section 328B has an axial length that is approximately 50% of the axial length "L+X+Y".

In the proximal section 326B of the tapered outer surface 322B, the outer geometry of the broach 302B defined by the stepped planes 314B is the same as the corresponding outer geometry of the sleeve 224B defined by the stepped walls 274B. In other words, the number of stepped planes 314B is equal to the number of stepped walls 274B, and each stepped plane 314B has the same maximum medial-lateral dimension, the same maximum anterior-posterior dimension, and the same axial height as the corresponding stepped wall 274B. As a result, the broach 302B is configured to define a cavity in the patient's femur that includes a proximal section that is substantially the same as the sleeve 224B such that the sleeve 224B is fitted into that section.

In the distal section 328B of the tapered outer surface 322B the number of stepped planes 314B is equal to the number of stepped walls 274B, and each stepped plane 314B has the same axial height as the corresponding stepped wall 274B. However, the maximum medial-lateral dimension and the maximum anterior-posterior dimension of each stepped plane 314B are smaller than the maximum medial-lateral dimension and the maximum anterior-posterior dimension of the corresponding stepped wall 274B. In other words, the outer geometry of the broach 302B defined by the stepped planes 314B is smaller than the corresponding outer geometry of the sleeve 224B defined by the stepped walls 274B. As a result, the broach 302B is configured to define a cavity in the patient's femur that includes a distal section that is smaller than the sleeve 224B such that the sleeve 224B is press fit into that section.

The maximum medial-lateral dimension of each stepped plane 314B in the distal section 328B is 0.35 mm less than the maximum medial-lateral dimension of the corresponding stepped wall 274B of the sleeve 224B. Similarly, the maximum anterior-posterior dimension of each stepped plane 314B in the distal section 328B is 0.35 mm less than the maximum anterior-posterior dimension of the corresponding stepped wall 274B of the sleeve 224B. It should be appreciated that the dimensions of the broach 302B may be adjusted to provide greater or less press fit for the sleeve 224B.

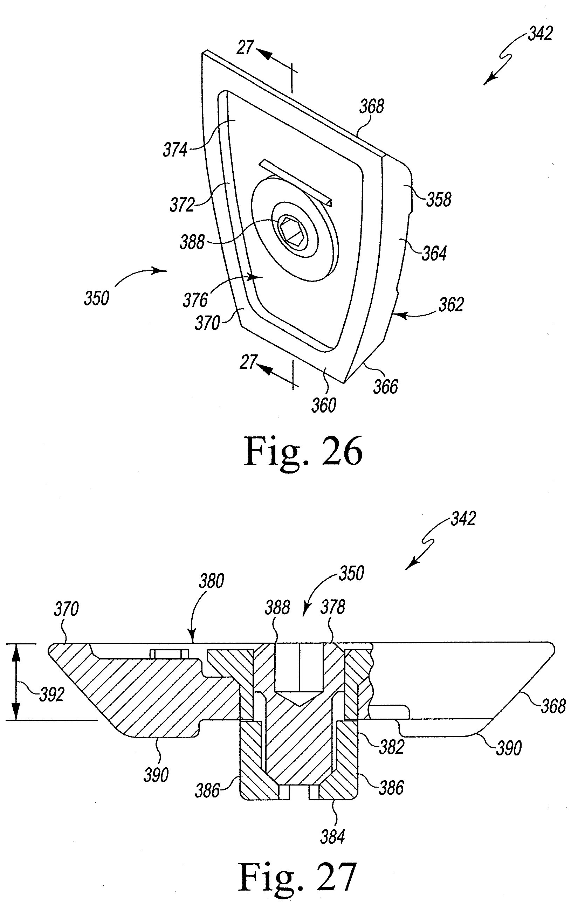

FIGS. 26 to 35 show a plurality of augments 340 of the femoral components 200. The augments 340 include a plurality of distal augments 342, 344 (see FIGS. 26 to 29) and a plurality of posterior augments 346, 348. As described above, each of the augments 340 includes a mounting plug 256 that is configured to be received in the mounting aperture 250. Each augment 340 also includes a retention mechanism 350 configured secure the corresponding augment 340 to the femoral component 210, as described in greater detail below. The augments 340 are formed from any suitable implant-grade metallic material such as, for example, cobalt-chromium, titanium, or stainless steel.

As shown in FIGS. 26 and 27, the distal augment 342 includes a wedge-shaped body 358 that has a proximal surface 360, a distal surface 362 positioned opposite the proximal surface 360, and a side wall 364 that connects the surfaces 360, 362. The side wall 364 includes a tapered anterior surface 366 and a tapered posterior surface 368, which extend obliquely relative the surfaces 360, 362. When the distal augment 342 is secured to the femoral component 210, the tapered anterior surface 366 is configured to engage the anterior chamfer surface 236 of the femoral component 210, and the tapered posterior surface 368 is configured to engage the posterior chamfer surface 234 of the femoral component 210.

As shown in FIG. 26, the proximal surface 360 of the distal augment 342 has a rim surface 370 and a side wall 372 that extends inwardly from the rim surface 370. The side wall 372 cooperates with a bottom surface 374 to define a pocket 376 in the proximal surface 360. The upper end 378 of the mounting plug 256 is positioned an opening 380 defined in the bottom surface 374, and the body 382 of the plug 256 extends through the augment body 358 to an end 384 positioned below the body 358, as shown in FIG. 27. The end 384 of the body 382 is divided into four legs 386.

The retention mechanism 350 of the augment 340 includes a fastener 388 that is threaded into the body 382 of the mounting plug 256. The fastener 288 includes a socket in which a driver may be inserted to rotate the fastener 288. When the fastener 388 is rotated in a first direction, the fastener 388 is driven toward the end 384 of the body 382, causing the legs 386 to expand outward; when the fastener is rotated in the opposite direction, the fastener 388 moves away from the end 384 of the body 382 such that the legs 386 are permitted to retract.

The distal surface 362 of the wedge-shaped body 358 is configured to engage the distal surface 230 of the femoral component 210. A plurality of feet 390 extend from the distal surface 362 of the wedge-shaped body 358. Each foot 390 is sized to be positioned in the cement pocket 240 of the femoral component 210. As shown in FIG. 27, the wedge-shaped body 358 also has a thickness 392 defined between the distal surface 362 and the proximal surface 360.

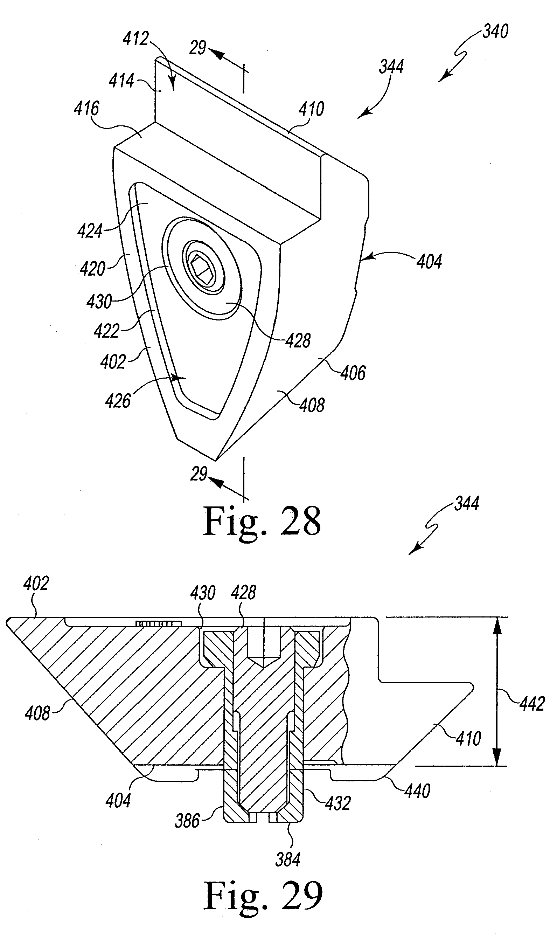

As shown in FIGS. 28 and 29, the distal augment 344 includes a wedge-shaped body 400 that has a proximal surface 402, a distal surface 404 positioned opposite the proximal surface 402, and a side wall 406 that connects the surfaces 402, 404. The side wall 406 includes a tapered anterior surface 408 and a tapered posterior surface 410, which extend obliquely relative the surfaces 408, 410. When the distal augment 344 is secured to the femoral component 210, the tapered anterior surface 408 is configured to engage the anterior chamfer surface 236 of the femoral component 210, and the tapered posterior surface 410 is configured to engage the posterior chamfer surface 234 of the femoral component 210.

The side wall 406 has a posterior notch 412 defined therein. As shown in FIGS. 28 and 29, the notch 412 is defined by a substantially planar proximal surface 414 extending parallel to the proximal surface 402 and anteriorly from an edge of the tapered posterior surface 410 and a substantially planar posterior surface 416 extending orthogonal to the proximal surface 414.

The notch 412 is sized to receive the posterior augment 348, as described in greater detail below.

As shown in FIG. 28, the proximal surface 402 of the distal augment 344 has a configuration similar to the proximal surface 360 of the distal augment 342. The surface 402 has a rim surface 420 and a side wall 422 that extends inwardly from the rim surface 420. The side wall 422 cooperates with a bottom surface 424 to define a pocket 426 in the proximal surface 402. The upper end 428 of the mounting plug 256 is positioned an opening 430 defined in the bottom surface 424, and the body 432 of the plug 256 extends through the augment body 358 to an end 384 positioned below the body 358, as shown in FIG. 29. The end 384 of the body 432 is divided into four legs 386. The retention mechanism 350 of the augment 340 includes a fastener 438 that is threaded into the body 432 of the mounting plug 256. The fastener 438 includes a socket in which a driver may be inserted to rotate the fastener 438. When the fastener 438 is rotated in a first direction, the fastener 438 is driven toward the end 384 of the body 432, causing the legs 386 to expand outward; when the fastener is rotated in the opposite direction, the fastener 438 moves away from the end 384 of the body 432 such that the legs 386 are permitted to retract.

The distal surface 404 of the wedge-shaped body 400 is configured to engage the distal surface 230 of the femoral component 210. A plurality of feet 440 extend from the distal surface 404 of the wedge-shaped body 400. Each foot 440 is sized to be positioned in the cement pocket 240 of the femoral component 210. As shown in FIG. 29, the wedge-shaped body 358 also has a thickness 442 defined between the distal surface 404 and the proximal surface 402.

As shown in FIGS. 27 and 29, the thickness 442 of the augment 344 is greater than the thickness 392 of the augment 342. The thickness 392 is equal to approximately 4 mm, and the thickness 442 is equal to approximately 12 mm. The thicknesses of the augments may be more or less than these values depending with the size of the other femoral components 200. Additionally, as shown in FIGS. 27 and 29, the distal surface 404 of the augment 344 is wider than distal surface 362 of the augment 342.

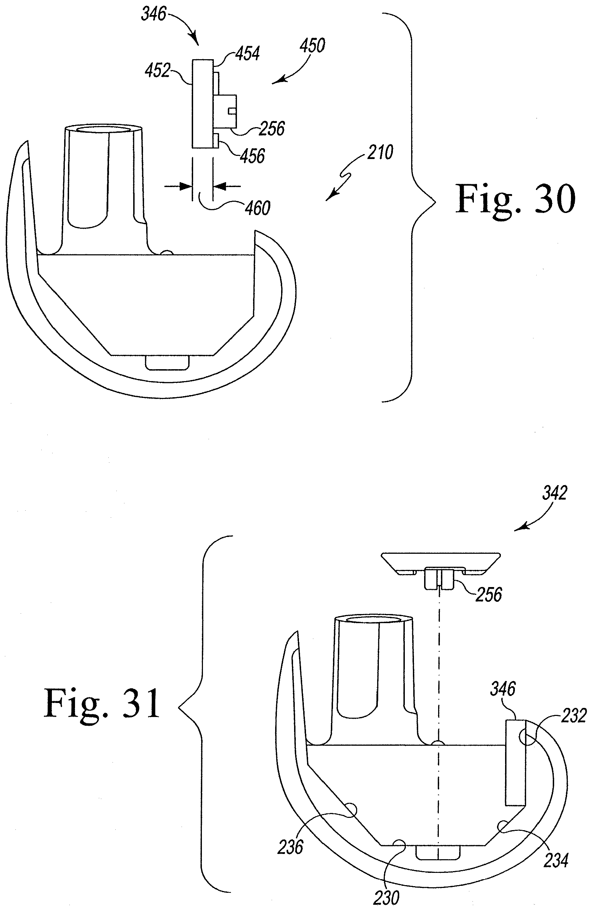

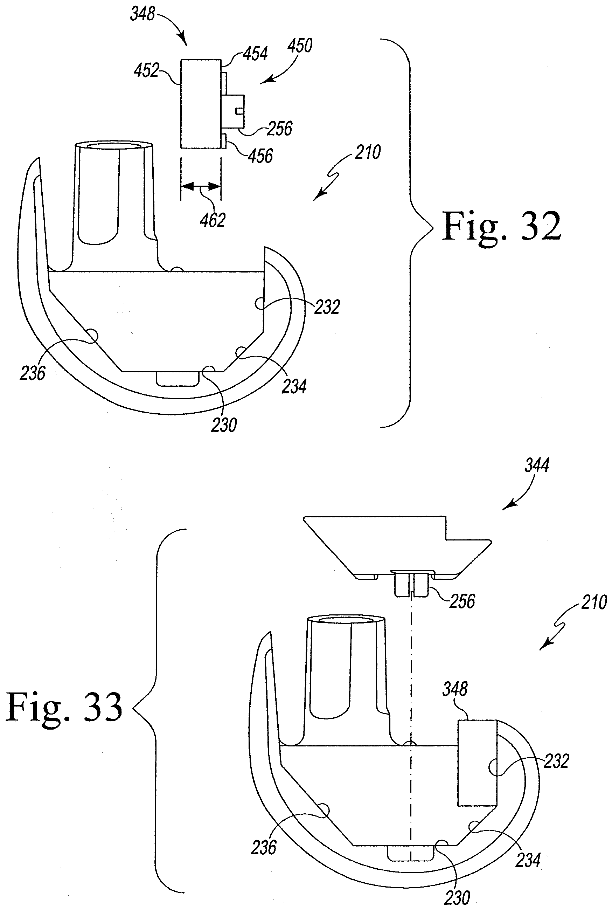

As described above, the femoral components 200 also include posterior augments 346, 348. Each of the posterior augments 346, 348 includes a body 450 having an anterior surface 452 and a posterior surface 454 positioned opposite the anterior surface 452. As shown in FIGS. 30 and 32, each of the posterior augments 346, 348 includes the mounting plug 256, which has a configuration similar to the configurations described above with reference to the distal augments 342, 344.

The posterior surface 454 of the body 450 is configured to engage the posterior fixation surface 232 of the femoral component 210. A plurality of feet 456 extend from the posterior surface 454. Each foot 456 is sized to be positioned in the cement pocket 240 of the femoral component 210. As shown in FIG. 30, the posterior augment 346 has a thickness 460 defined between the anterior surface 452 and the posterior surface 454; as shown in FIG. 32, the posterior augment 348 has a thickness 462 defined between the anterior surface 452 and the posterior surface 454. The thickness 462 of the augment 348 is greater than the thickness 460 of the augment 346.

In use, the augments 340 maybe attached to the femoral component 210 in the same sequence, regardless of the combination of augments 340 used. For example, as shown in FIGS. 30 and 31, the posterior augment 346 maybe attached first to the posterior fixation surface 232 of the femoral component 210 via the mounting plug 256, which is inserted into the aperture 260. Using the fastener (not shown) of the mounting plug 256, the legs 386 of the mounting plug 256 are expanded into engagement with the wall 252 defining the aperture 250, thereby securing the augment 346 to the posterior fixation surface 232.

The distal augment 342 may then be attached to the distal fixation surface 230. As shown in FIG. 31, the mounting plug 256 is aligned with the aperture 250 of the distal fixation surface 230. The distal augment 342 may be advanced downward such that the plug 256 is received in the aperture 250. The fastener 388 may then be operated to engage the wall 252 with the legs 386 of the mounting plug 256, thereby securing the augment 342 to the distal fixation surface 230.

As shown in FIGS. 32 to 35, another combination of augments 340 (in this case, the largest augments 344, 348) may be attached in the same sequence as the augments 342, 346. To do so, the posterior augment 348 may be attached first to the posterior fixation surface 232 of the femoral component 210 by means of the mounting plug 256, which is inserted into the aperture 260. Using the fastener (not shown) of the mounting plug 256, the legs 386 of the mounting plug 256 are expanded into engagement with the wall 252 defining the aperture 250, thereby securing the augment 348 to the posterior fixation surface 232.

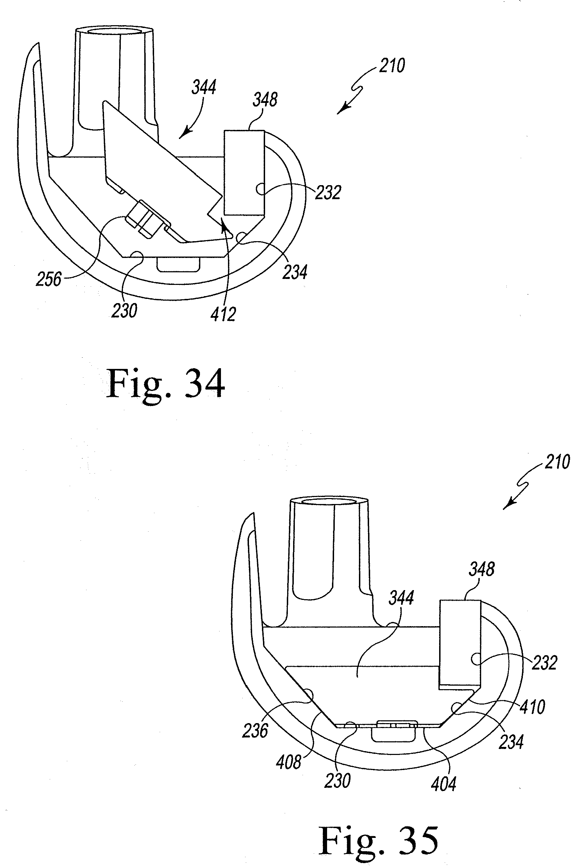

The distal augment 344 may then be attached to the distal fixation surface 230. To do so, the distal augment 344 is positioned above the distal fixation surface 230 as shown in FIG. 33. The augment 344 may then be rotated as shown in FIG. 34 and advanced downward.

As shown in FIG. 34, the user may slide posterior edge of the distal augment 344 under the posterior augment 348 to "hook" the distal augment 344 into position. In doing so, the posterior augment 348 is advanced into the posterior notch 412 of the distal augment 344. When the plug 256 of the distal augment 344 is received in the aperture 250 and the augment 344 is properly seated as shown in FIG. 35, the posterior augment 348 remains in the posterior notch 412. The fastener 388 may then be operated to engage the wall 252 with the legs 386 of the mounting plug 256, thereby securing the augment 348 to the distal fixation surface 230.

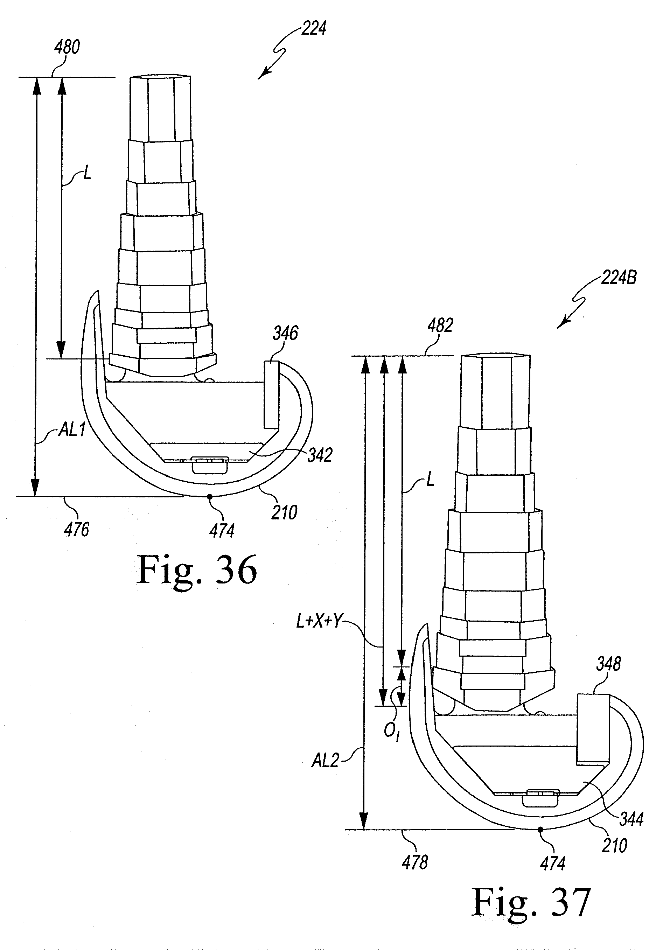

As shown in FIGS. 36 and 37, the femoral components 200 may be assembled to form a femoral orthopaedic prosthesis. In FIG. 36, the smallest femoral sleeve 224 and the smallest augments 342, 346 are assembled with the femoral component 210 to form prosthesis 470. In FIG. 37, the largest femoral sleeve 224B and the largest augments 344, 348 are assembled with the femoral component 210 to form prosthesis 472. As shown in FIGS. 36-37, the distalmost points 474 of the condyle surfaces 12, 14 define a joint line of the femoral orthopaedic prosthesis when the patient's leg is extension. In FIG. 36, the joint line is indicated by line 476; in FIG. 37, the joint line is indicated by line 478. The assemblies have maximum axial lengths from planes at the proximal ends 266, 266B (the planes shown at 480 and 482) to the plane of the joint line, shown at 476 in FIG. 36 and at 478 in FIG. 37. These maximum axial lengths of the assemblies are shown at AL1 in FIG. 36 and AL2 in FIG. 37. In the device shown in the drawings, AL2 is longer than AL1 by the dimension "X+Y", that is the axial length of the sleeve 224B beyond the length "L" of the smallest sleeve 224.

As can also be seen from a comparison of FIGS. 36 and 37, using the larger sleeve 224B distalizes the joint line 476 to the position 478 by the offset distance o₁. This offset distance o₁ also corresponds with the dimension "X+Y". Similarly, using the sleeve 224A distalizes the joint line by the dimension "X" relative to the joint line 476.

Since the geometries of the stepped bodies 264, 264B, 264C of the different sizes of sleeves 224, 224A, 224B are the same through axial length "L", the surgeon can prepare the distal femur using the broach 302 to receive the smallest size of femoral sleeve 224. If the surgeon determines intraoperatively that the joint line should be distalized, the surgeon may use any of the other sizes of sleeve 224A, 224B, and the proximal portion of the larger size sleeve will fit within the opening prepared in the femur to receive the smaller sleeve and extend distally from the bone by the distance "X" or "X+Y" thereby to offset the joint line distally. The thickness 442 of the distal augment 344 is equal to the offset distance o₁ such that the sleeve 224B the prosthesis 472 may be stabilized when the joint line is distalized. The surgeon can therefore accomplish this distalization without any further preparation of the bone cavity.

Another system providing the option of distalizing the joint line is disclosed in EP-A-2710979.

## Claims

1. An orthopaedic knee prosthesis system, comprising:
a prosthetic knee component (10) configured to be implanted into the distal end of a patient's femur and having medial and lateral condyle surfaces (12, 14),
a first sleeve component (24) including (i) a first end (56) configured to be separately secured to the prosthetic knee component and (ii) a tapered section (49; L) extending from the first end to a second end (26) positioned opposite the first end, and
a second sleeve component (24A) including (i) a first end (56A) configured to be separately secured to the prosthetic knee component in place of the first sleeve component, (ii) a first tapered section (49A; L) extending distally from a second end (26A) positioned opposite the first end, and (iii) a second tapered section (49A; X) extending distally from the first tapered section,
**characterised in that** the tapered section (49; L) of the first sleeve component (24) and the first tapered section (49A; L) of the second sleeve component (26) have a first outer geometry and the second tapered section (49A; X) has a second outer geometry different from the first outer geometry.

2. The orthopaedic knee prosthesis system of claim 1, in which the first outer geometry is defined by a plurality of stepped walls (274) and a plurality of annular walls (276), each annular wall connecting a pair of stepped walls of the plurality of stepped walls.

3. The orthopaedic knee prosthesis system of claim 2, in which:
the plurality of stepped walls defining the first outer geometry is a first plurality of stepped walls (274; L), and
the second outer geometry is defined by a second plurality of stepped walls (274A;L) and a second plurality of annular walls (274A; X), each annular wall of the second plurality of annular walls connecting a pair of stepped walls of the second plurality of stepped walls.

4. The orthopaedic knee prosthesis system of claim 3, in which:
each stepped wall of the first plurality of stepped walls (274; L) has a maximum medial-lateral dimension,
each stepped wall of the second plurality of stepped walls (274A; X) has a maximum medial-lateral dimension, and
the maximum medial-lateral dimension of each stepped wall of the first plurality of stepped walls is less than the maximum medial-lateral dimension of each stepped wall of the second plurality of stepped walls.

5. The orthopaedic knee prosthesis system of claim 4, in which:
each stepped wall of the first plurality of stepped walls (274; L)has a maximum anterior-posterior dimension,
each stepped wall of the second plurality of stepped walls (274A; L) has a maximum anterior-posterior dimension, and
the maximum anterior-posterior dimension of each stepped wall of the first plurality of stepped walls is less than the maximum anterior-posterior dimension of each stepped wall of the second plurality of stepped walls.

6. The orthopaedic knee prosthesis system of claim 1, further comprising:
a third sleeve component (24B) which includes (i) a first end (56B) configured to be separately secured to the prosthetic knee component in place of the first sleeve component, (ii) a first tapered section (49B; L) extending distally from a second end (26B) positioned opposite the first end, (iii) a second tapered section (49B; X) extending distally from the first tapered section, and (iv) a third tapered section (49B; Y) extending distally from the first tapered section,
in which the second tapered section (49B; X) of the third sleeve component (24B) has the second outer geometry and the third tapered section (49B; Y) has a third outer geometry different from the first outer geometry and the second outer geometry.

7. The orthopaedic knee prosthesis system of claim 6, in which:
the tapered section (49; L) of the first sleeve component (24) defines a maximum axial length (L) of the first sleeve component,
the first tapered section (49A; L) and the second tapered section (49A; X)of the second sleeve component (24A) define a maximum axial length (L+X) of the second sleeve component that is greater than the maximum axial length of the first sleeve component,
the first tapered section (49B; L), the second tapered section (49B; X), and the third tapered section (49B; Y) of the third sleeve component (24B) define a maximum axial length (L+X+Y) of the third sleeve component that is greater than the maximum axial length of the second sleeve component.

8. The orthopaedic knee prosthesis system of claim 6, in which the first tapered section (49B; L) of the third sleeve component (24B) has the first outer geometry.

## Patentansprüche

1. Orthopädisches Knieprothesensystem, umfassend:
eine prothetische Kniekomponente (10), die ausgestaltet ist, in das distale Ende des Oberschenkelknochens eines Patienten implantiert zu werden und mediale und laterale Kondylusflächen (12, 14) aufweist,
eine erste Hülsenkomponente (24) mit (i) einem ersten Ende (56), das ausgestaltet ist, an der prothetischen Kniekomponente separat fixiert zu werden, und (ii) einem konisch zulaufenden Abschnitt (49; L), der sich von dem ersten Ende zu einem zweiten Ende (26) erstreckt, das gegenüber dem ersten Ende positioniert ist, und
eine zweite Hülsenkomponente (24A) mit (i) einem ersten Ende (56A), das ausgestaltet ist, an der prothetischen Kniekomponente anstelle der ersten Hülsenkomponente separat fixiert zu werden, (ii) einem ersten konisch zulaufenden Abschnitt (49A; L), der sich distal von einem zweiten Ende (26A) erstreckt, das gegenüber dem ersten Ende positioniert ist, und (iii) einem zweiten konisch zulaufenden Abschnitt (49A; X), der sich distal von dem ersten konisch zulaufenden Abschnitt erstreckt,
**dadurch gekennzeichnet, dass** der konisch zulaufende Abschnitt (49; L) der ersten Hülsenkomponente (24) und der erste konisch zulaufende Abschnitt (49A; L) der zweiten Hülsenkomponente (26) eine erste äußere Geometrie aufweisen und der zweite konisch zulaufende Abschnitt (49A; X) eine zweite äußere Geometrie aufweist, die sich von der ersten äußeren Geometrie unterscheidet.

2. Orthopädisches Knieprothesensystem nach Anspruch 1, wobei die erste äußere Geometrie durch eine Vielzahl von stufenförmigen Wänden (274) und eine Vielzahl von ringförmigen Wänden (276) bestimmt wird, wobei jede ringförmige Wand ein Paar stufenförmiger Wände aus der Vielzahl von stufenförmigen Wänden verbindet.

3. Orthopädisches Knieprothesensystem nach Anspruch 2, wobei:
die Vielzahl von stufenförmigen Wänden, die die erste äußere Geometrie bestimmt, eine erste Vielzahl von stufenförmigen Wänden (274; L) ist, und
die zweite äußere Geometrie durch eine zweite Vielzahl von stufenförmigen Wänden (274A; L) und eine zweite Vielzahl von ringförmigen Wänden (274A; X) bestimmt wird, wobei jede ringförmige Wand der zweiten Vielzahl von ringförmigen Wänden ein Paar stufenförmiger Wände aus der zweiten Vielzahl von stufenförmigen Wänden verbindet.

4. Orthopädisches Knieprothesensystem nach Anspruch 3, wobei:
jede stufenförmige Wand der ersten Vielzahl von stufenförmigen Wänden (274; L) eine maximale mediolaterale Dimension aufweist,
jede stufenförmige Wand der zweiten Vielzahl von stufenförmigen Wänden (274A; X) eine maximale mediolaterale Dimension aufweist, und
die maximale mediolaterale Dimension jeder stufenförmigen Wand der ersten Vielzahl von stufenförmigen Wänden geringer ist als die maximale mediolaterale Dimension jeder stufenförmigen Wand der zweiten Vielzahl von stufenförmigen Wänden.

5. Orthopädisches Knieprothesensystem nach Anspruch 4, wobei:
jede stufenförmige Wand der ersten Vielzahl von stufenförmigen Wänden (274; L) eine maximale anteroposteriore Dimension aufweist,
jede stufenförmige Wand der zweiten Vielzahl von stufenförmigen Wänden (274A; L) eine maximale anteroposteriore Dimension aufweist, und
die maximale anteroposteriore Dimension jeder stufenförmigen Wand der ersten Vielzahl von stufenförmigen Wänden geringer ist als die maximale anteroposteriore Dimension jeder stufenförmigen Wand der zweiten Vielzahl von stufenförmigen Wänden.

6. Orthopädisches Knieprothesensystem nach Anspruch 1, ferner umfassend:
eine dritte Hülsenkomponente (24B), die (i) ein erstes Ende (56B) umfasst, das ausgestaltet ist, an der prothetischen Kniekomponente anstelle der ersten Hülsenkomponente separat fixiert zu werden, (ii) einem ersten konisch zulaufenden Abschnitt (49B; L), der sich distal von einem zweiten Ende (26B) erstreckt, das gegenüber dem ersten Ende positioniert ist, (iii) einem zweiten konisch zulaufenden Abschnitt (49B; X), der sich distal von dem ersten konisch zulaufenden Abschnitt erstreckt, und (iv) einem dritten konisch zulaufenden Abschnitt (49B; Y), der sich distal von dem ersten konisch zulaufenden Abschnitt erstreckt,
wobei der zweite konisch zulaufende Abschnitt (49B; X) der dritten Hülsenkomponente (24B) die zweite äußere Geometrie aufweist und der dritte konisch zulaufende Abschnitt (49B; Y) eine dritte äußere Geometrie aufweist, die sich von der ersten äußeren Geometrie und der zweiten äußeren Geometrie unterscheidet.

7. Orthopädisches Knieprothesensystem nach Anspruch 6, wobei:
der konisch zulaufende Abschnitt (49; L) der ersten Hülsenkomponente (24) eine maximale axiale Länge (L) der ersten Hülsenkomponente bestimmt,
der erste konisch zulaufende Abschnitt (49A; L) und der zweite konisch zulaufende Abschnitt (49A; X) der zweiten Hülsenkomponente (24A) eine maximale axiale Länge (L+X) der zweiten Hülsenkomponente bestimmen, die größer ist als die maximale axiale Länge der ersten Hülsenkomponente,
der erste konisch zulaufende Abschnitt (49B; L), der zweite konisch zulaufende Abschnitt (49B; X) und der dritte konisch zulaufende Abschnitt (49B; Y) der dritten Hülsenkomponente (24B) eine maximale axiale Länge (L+X+Y) der dritten Hülsenkomponente bestimmen, die größer ist als die maximale axiale Länge der zweiten Hülsenkomponente.

8. Orthopädisches Knieprothesensystem nach Anspruch 6, wobei der erste konisch zulaufende Abschnitt (49B; L) der dritten Hülsenkomponente (24B) die erste äußere Geometrie aufweist.

## Revendications

1. Système de prothèse de genou orthopédique :
un composant de genou prothétique (10) configuré pour être implanté dans l'extrémité distale du fémur d'un patient et ayant des surfaces condyliennes médiale et latérale (12, 14),
un premier composant de manchon (24) comprenant (i) une première extrémité (56) configurée pour être fixée séparément au composant de genou prothétique et (ii) une section progressivement rétrécie (49 ; L) s'étendant de la première extrémité à une deuxième extrémité (26) positionnée à l'opposé de la première extrémité, et
un deuxième composant de manchon (24A) comprenant (i) une première extrémité (56A) configurée pour être fixée séparément au composant de genou prothétique à la place du premier composant de manchon, (ii) une première section progressivement rétrécie (49A ; L) s'étendant de manière distale à partir d'une deuxième extrémité (26A) positionnée à l'opposé de la première extrémité, et (iii) une deuxième section progressivement rétrécie (49A ; X) s'étendant de manière distale à partir de la première section progressivement rétrécie,
**caractérisé en ce que** la section progressivement rétrécie (49 ; L) du premier composant de manchon (24) et la première section progressivement rétrécie (49A ; L) du deuxième composant de manchon (26) ont une première géométrie externe et la deuxième section progressivement rétrécie (49A ; X) a une deuxième géométrie externe différente de la première géométrie externe.

2. Système de prothèse de genou orthopédique selon la revendication 1, dans lequel la première géométrie externe est définie par une pluralité de parois étagées (274) et une pluralité de parois annulaires (276), chaque paroi annulaire connectant une paire de parois étagées de la pluralité de parois étagées.

3. Système de prothèse de genou orthopédique selon la revendication 2, dans lequel :
la pluralité de parois étagées définissant la première géométrie externe est une première pluralité de parois étagées (274 ; L), et
la deuxième géométrie externe est définie par une deuxième pluralité de parois étagées (274A ; L) et une deuxième pluralité de parois annulaires (274A ; X), chaque paroi annulaire de la deuxième pluralité de parois annulaires connectant une paire de parois étagées de la deuxième pluralité de parois étagées.

4. Système de prothèse de genou orthopédique selon la revendication 3, dans lequel :
chaque paroi étagée de la première pluralité de parois étagées (274 ; L) a une dimension médiale-latérale maximum ;
chaque paroi étagée de la deuxième pluralité de parois étagées (274A ; X) a une dimension médiale-latérale maximum, et
la dimension médiale-latérale maximum de chaque paroi étagée de la première pluralité de parois étagées est inférieure à la dimension médiale-latérale maximum de chaque paroi étagée de la deuxième pluralité de parois étagées.

5. Système de prothèse de genou orthopédique selon la revendication 4, dans lequel :
chaque paroi étagée de la première pluralité de parois étagées (274 ; L) a une dimension antérieure-postérieure maximum,
chaque paroi étagée de la deuxième pluralité de parois étagées (274A ; L) a une dimension antérieure-postérieure maximum, et
la dimension antérieure-postérieure maximum de chaque paroi étagée de la première pluralité de parois étagées est inférieure à la dimension antérieure-postérieure maximum de chaque paroi étagée de la deuxième pluralité de parois étagées.

6. Système de prothèse de genou orthopédique selon la revendication 1, comprenant en outre :
un troisième composant de manchon (24B) qui comprend (i) une première extrémité (56B) configurée pour être fixée séparément sur le composant de genou prothétique à la place du premier composant de manchon, (ii) une première section progressivement rétrécie (49B ; L) s'étendant de manière distale à partir d'une deuxième extrémité (26B) positionnée à l'opposé de la première extrémité, (iii) une deuxième section progressivement rétrécie (49B ; X) s'étendant de manière distale à partir de la première section étagée, et (iv) une troisième section progressivement rétrécie (49B ; Y) s'étendant de manière distale à partir de la première section progressivement rétrécie,
dans lequel la deuxième section progressivement rétrécie (49B ; X) du troisième composant de manchon (24B) a la deuxième géométrie externe et la troisième section progressivement rétrécie (49B ; Y) a une troisième géométrie externe différente de la première géométrie externe et de la deuxième géométrie externe.

7. Système de prothèse de genou orthopédique selon la revendication 6, dans lequel :
la section progressivement rétrécie (49 ; L) du premier composant de manchon (24) définit une longueur axiale maximum (L) du premier composant de manchon,
la première section progressivement rétrécie (49A ; L) et la deuxième section progressivement rétrécie (49A ; X) du deuxième composant de manchon (24A) définissent une longueur axiale maximum (L+X) du deuxième composant de manchon qui est supérieure à la longueur axiale maximum du premier composant de manchon,
la première section progressivement rétrécie (49B ; L), la deuxième section progressivement rétrécie (49B ; X), et la troisième section progressivement rétrécie (49B ; Y) du troisième composant de manchon (24B) définissent une longueur axiale maximum (L + X + Y) du troisième composant de manchon qui est supérieure à la longueur axiale maximum du deuxième composant de manchon.

8. Système de prothèse de genou orthopédique selon la revendication 6, dans lequel la première section progressivement rétrécie (49B ; L) du troisième composant de manchon (24B) a la première géométrie externe.
